# EUROPEAN PATENT SPECIFICATION

(11) **EP 2 943 794 B1**
(45) Date of publication and mention of the grant of the patent: **06.11.2019**
(21) Application number: 14738144.6
(22) Date of filing: 13.01.2014
(51) Int. Cl.: G01N 33/574, G01N 33/53, G01N 33/48

(54) **BIOMARKER PANEL FOR PREDICTING RECURRECNE OF PROSTATE CANCER**
BIOMARKERPANEL ZUR VORHERSAGE DES WIEDERAUFTRETENS VON PROSTATAKREBS
PANNEAU BIOMARQUEUR POUR PRÉDIRE LA RÉCIDIVE DU CANCER DE LA PROSTATE

(30) Priority: 13.01.2013 US 201361752135 P; 22.04.2013 US 201361814480 P; 17.09.2013 US 201361878648 P
(43) Date of publication of application: 18.11.2015
(73) Proprietor: Emory University, Atlanta, GA 30322 (US)
(72) Inventor: MORENO, Carlos, Sanchez, Atlanta, GA 30327 (US); LONG, Qi, Atlanta, GA 30329 (US)
(74) Representative: Lau, Sarah Jane
(86) International application number: PCT/US2014/011211
(87) International publication number: WO 2014/110475

(56) References cited:
- WO-A1-00/20869
- WO-A1-2012/122626
- WO-A2-02/31209
- WO-A2-2006/091776
- WO-A2-2013/006495
- US-A1- 2007 237 770
- US-A1- 2009 170 715
- US-A1- 2010 233 691
- M. A. TARIQ ET AL: "Whole-transcriptome RNAseq analysis from minute amount of total RNA", NUCLEIC ACIDS RESEARCH, vol. 39, no. 18, 1 October 2011 (2011-10-01), pages e120-e120, XP055280292, GB ISSN: 0305-1048, DOI: 10.1093/nar/gkr547
- HENRIK STRANNEHEIM ET AL: "Scalable Transcriptome Preparation for Massive Parallel Sequencing", PLOS ONE, vol. 6, no. 7, 7 July 2011 (2011-07-07), page e21910, XP055280253, DOI: 10.1371/journal.pone.0021910
- Illumina: "TruSeq Stranded mRNA and Total RNA Sequencing Sample Preparation Solutions", , 18 September 2012 (2012-09-18), XP055280331, Retrieved from the Internet: URL:http://www.illumina.com/company/news-c enter/press-releases/press-release-details .html?newsid=1735908 [retrieved on 2016-06-14]
- J CUZICK ET AL: "Prognostic value of an RNA expression signature derived from cell cycle proliferation genes in patients with prostate cancer: a retrospective study", THE LANCET, vol. 12, no. 3, 1 March 2011 (2011-03-01), pages 245-255, XP055275747, DOI: 10.1016/S1470-
- QI LONG ET AL: "Protein-Coding and MicroRNA Biomarkers of Recurrence of Prostate Cancer Following Radical Prostatectomy", AMERICAN JOURNAL OF PATHOLOGY., vol. 179, no. 1, 1 July 2011 (2011-07-01), pages 46-54, XP055262128, US ISSN: 0002-9440, DOI: 10.1016/j.ajpath.2011.03.008
- VARAMBALLY S ET AL: "Integrative genomic and proteomic analysis of prostate cancer reveals signatures of metastatic progression", CANCER CELL, CELL PRESS, US, vol. 8, no. 5, 14 November 2005 (2005-11-14), pages 393-406, XP002395335, ISSN: 1535-6108, DOI: 10.1016/J.CCR.2005.10.001
- TOHRU NAKAGAWA ET AL: "A Tissue Biomarker Panel Predicting Systemic Progression after PSA Recurrence Post-Definitive Prostate Cancer Therapy", PLOS ONE, vol. 3, no. 5, 28 May 2008 (2008-05-28), page e2318, XP055010915, DOI: 10.1371/journal.pone.0002318
- DAVID B ALLISON ET AL: "Microarray data analysis: from disarray to consolidation and consensus", NATURE REVIEWS: GENETICS, NATURE PUBLISHING GROUP, UNITED KINGDOM, vol. 7, no. 1, 1 January 2006 (2006-01-01) , pages 55-65, XP009100551, ISSN: 1471-0064, DOI: 10.1038/NRG1749
- BARRY S. TAYLOR ET AL: "Integrative Genomic Profiling of Human Prostate Cancer", CANCER CELL, vol. 18, no. 1, 1 July 2010 (2010-07-01), pages 11-22, XP055280056, US ISSN: 1535-6108, DOI: 10.1016/j.ccr.2010.05.026
- LONG ET AL.: 'Protein-coding and microRNA biomarkers of recurrence of prostate cancer following radical prostatectomy' THE AMERICAN JOURNAL OF PATHOLOGY vol. 179, no. 1, 03 May 2011, pages 46 - 54, XP055262128
- CALVO ET AL.: 'Prostate cancer and the genomic revolution: advances using microarray analyses' MUTATION RESEARCH vol. 576, no. 1-2, 25 August 2005, pages 66 - 79, XP027632641

## Description

### GOVERNMENT SUPPORT

This invention was made with government support under Grant No. W81XWH-10-1-0090 awarded by the Department of Defense, Grants R01CA106826, U01 CA168449, R03CA173770, R03CA183006, R01CA128813 awarded by the National Institute of Health, and Grant No. P30CA138292 awarded by the National Cancer Institute. The government has certain rights in the invention.

### FIELD

This invention generally relates to 24 biomarkers for prostate cancer methods, and kits related thereto.

### BACKGROUND

The widespread use of screening with prostate specific antigen (PSA) has resulted in both increased use of prostate biopsy and incidence of diagnosed prostate cancer. While results of the Prostate, Lung, Colorectal, and Ovarian (PLCO) Cancer Screening Trial showed no reduction in prostate cancer specific mortality when comparing systematic annual PSA screening to opportunistic screening, the European Randomized Study of Screening for Prostate Cancer (ERPSC) found a small reduction in mortality (1 death per 1000 men screened) in a PSA-screening naive population. Several potential flaws in the ERPSC study and the associated harms of over diagnosis and over treatment led the US Preventive Services Task Force (USPSTF) to recommend against PSA screening. Recent changes in recommendations regarding PSA screening notwithstanding, the number of prostate biopsies performed in the U.S. each year is significant. For men over age 65 who are Medicare beneficiaries, more than one million prostate biopsies are performed annually.

Results from the Prostate Cancer Intervention Versus Observation Trial (PIVOT) trial indicate that radical prostatectomy reduces mortality for patients with high-risk PCa (PSA> 10 ng/ml) compared to "active surveillance" (AS), in which patients are monitored by following a prescribed protocol including repeat PSA and prostate biopsy at predetermined intervals, although there was no benefit for low-risk cases. AS is considered most appropriate for men with low-risk cancers or with a life expectancy <10 years. Those men with high- or very-high risk cancers or longer life expectancy may benefit from a more aggressive therapy given at time of diagnosis such as radical prostatectomy (RP), external beam radiation therapy, or radioactive seed implant (brachytherapy). However, urinary incontinence and sexual dysfunction are common following surgery and radiation for prostate cancer. These side effects impact health-related quality of life and may be long-term. Since the prostate cancers being treated with these aggressive therapies are often indolent and thus may not impact the man's life or health, the risk/benefit ratio of various treatments may need careful consideration by the patient and family. It is estimated that about 50% of men who are diagnosed with prostate cancer as a result of PSA testing would remain asymptomatic and not require treatment. Yet, up to 90% of these men receive curative therapy: either surgery or radiation. Thus, there is a need for robust biomarkers to predict which tumors are more likely to result in different clinical outcomes for optimizing treatment decisions.

J Cuzick et al., ("Prognostic value of an RNA expression signature derived from cell cycle proliferation genes in patients with prostate cancer: a retrospective study", Lancet Oncol., Vol. 12, no. 3, 1 March 2011) discloses a panel of 31 cell cycle progression genes for predicting the biochemical recurrence of prostate cancer.

Qi Long et al., ("Protein-Coding and MicroRNA Biomarkers of Recurrence of Prostate Cancer Following Radical Prostatectomy", American Journal of Pathology, Vol. 179, no. 1, 1 July 2011) discloses protein-coding and microRNA biomarkers of recurrence of prostate cancer following radical prostatectomy.

WO 2012/122626 discloses marker sets for predicting prostate cancer prognosis.

WO 2006/091776 also discloses the use of gene expression profiles for predicting prognosis of a subject diagnosed with prostate cancer.

US 2010/233691 discloses methods of assessing or monitoring the response to therapy in a subject having prostate cancer with high accuracy, comprising assessing genes in a set of marker genes.

### SUMMARY

The protection sought for this invention is as defined in the claims.

In a first aspect, the invention relates to a method of determining the risk of recurrence of prostate cancer in a subject, comprising
analyzing a prostatectomy sample from a subject to determine RNA levels of biomarkers BTG2, CDC37L1, COL15A1, COL3A1, EIF2D, FDPS, HIST1H1C, HIST1H2BG, IFT57, IGFBP3, ITPR1, LBH, LOC284801, MARCH5, MED4, MEMO1, MXI1, PTN, RPL23AP53, SACM1L, SIRT1, SNORA20, SRSF3, and SYNM in the sample, and
comparing the levels of all of the biomarkers in the sample to prostate cancer-positive and/or prostate cancer-negative reference levels of the biomarkers, wherein
levels of the biomarkers in the sample matching the prostate cancer-positive reference levels, or being differentially present compared to the prostate cancer-negative reference levels, are indicative of the subject being predisposed to recurrence of prostate cancer, and wherein levels of the biomarkers in the sample matching the prostate cancer-negative reference levels, or being differentially present compared to the cancer-positive reference levels, are indicative of the subject not being predisposed to recurrence of prostate cancer.

The method may further comprise recording the measurements or comparisons of the biomarkers on a computer readable medium.

In a second aspect, the invention relates to a kit comprising nucleic acids configured to bind 24 RNA biomarkers for cancer, wherein the RNA biomarkers are BTG2, CDC37L1, COL15A1, COL3A1, EIF2D, FDPS, HIST1H1C, HIST1H2BG, IFT57, IGFBP3, ITPR1, LBH, LOC284801, MARCH5, MED4, MEMO1, MXI1, PTN, RPL23AP53, SACM1L, SIRT1, SNORA20, SRSF3, and SYNM, and wherein the nucleic acids configured to bind the RNA biomarkers are covalently attached to an array.

The kit may further comprise one or more or all of the components selected from oligonucleotides or pairs configured to bind for copying predetermined nucleic acid sequences associated with the biomarkers, adaptor oligonucleotides configured to bind to oligonucleotide pairs, adaptor oligonucleotides configured to bind or hybridize to nucleic acids attached to an array, ligase, circularization ligase, polymerase, a mix of deoxynucleotides, a biotinylated nucleotide, and a streptavidin bead

In certain embodiments, the methods further comprise recording the measurements or comparisons of the biomarkers on a computer readable medium.

In some embodiments, the methods further comprise the step of recording the measurements or comparisons of the biomarkers. In some embodiments, the measurements or comparisons are recorded in an electronic form on a computer readable medium. In some embodiments, the methods further comprise the step of recording the diagnosis. In some embodiments, the methods further comprise the step of reporting the measurements, comparisons, or diagnosis to a medical professional, the subject, or representative thereof.

In certain aspects, the disclosure contemplates kit and arrays comprising biomarker binding molecules and probes to gene mutations disclosed herein.

### BRIEF DESCRIPTION OF THE DRAWINGS

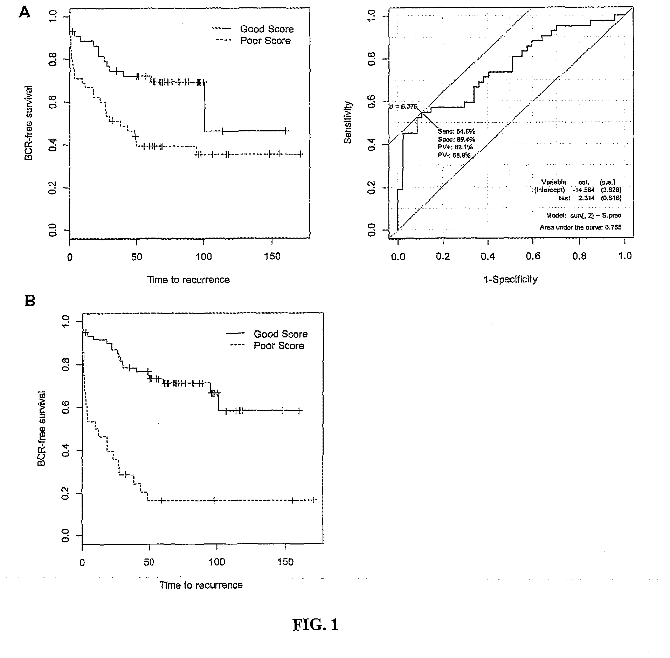

Figure 1 shows data for the biomarkers SYNM, GPX1, TMSB10, IFT57, ITPR1, PTN, and LAPTM5, A) Kaplan-meier curves for the two groups (log-rank test p= 0.000648) based on the prediction model using gene markers only. Using the coefficients and the optimal cut off of 0.225, the predictive scores were calcualted for all subjects divided into two groups. B) Kaplan-meier curves for the two groups (log-rank test p= 0.000592) based on the prediction model using both gene markers and clinical variables. Using the coefficients and the optimal cut off 3.426, the predictive scores were calculated for all subjects and divided into two groups.

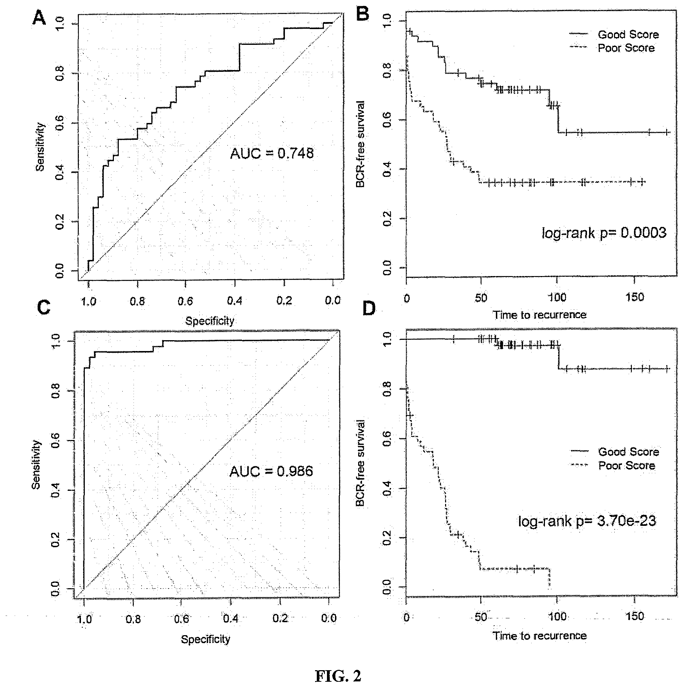

Figure 2 shows data of receiver-operator characteristic (ROC) and Kaplan-Meier (K-M) survival curves for two groups from 97 cases defined by clinical parameters alone (A-B) or clinical parameters combined with the biomarker genes described in Table 2 (C-D). (A) ROC curve using clinical parameters only (AUC = 0.75). (B) K-M curve of BCR-free survival using clinical parameters only. (C) ROC curve using biomarker score including clinical variables (AUC = 0.99). (D) K-M curve of BCR-free survival (log-rank test p=3.7e-23) including clinical variables.

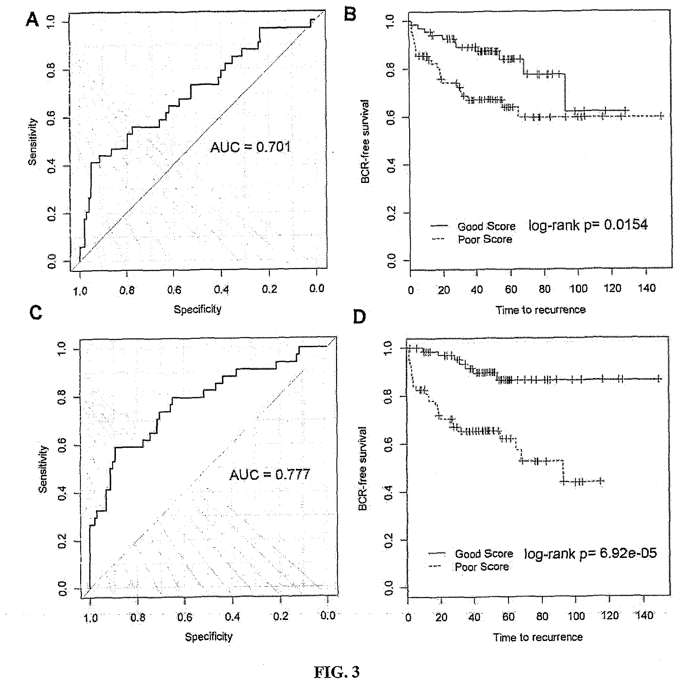

Figure 3 shows data on Receiver-operator characteristic (ROC) and Kaplan-Meier (K-M) survival curves for 140 cases from Taylor et al. using clinical parameters alone (A-B) or clinical parameters combined with the biomarker genes developed described in Table 2 (C-D). (A) ROC curve using clinical parameters only (AUC = 0.70). (B) K-M curve of BCR-free survival using clinical parameters (log-rank test p = 0.0154). (C) ROC curve using biomarkers from Table 2 plus clinical parameters (AUC = 0.78). (D) K-M curve of BCR-free survival for samples from Taylor et al. (log-rank test p= 0.6.9e-5) using RNA biomarkers plus clinical parameters.

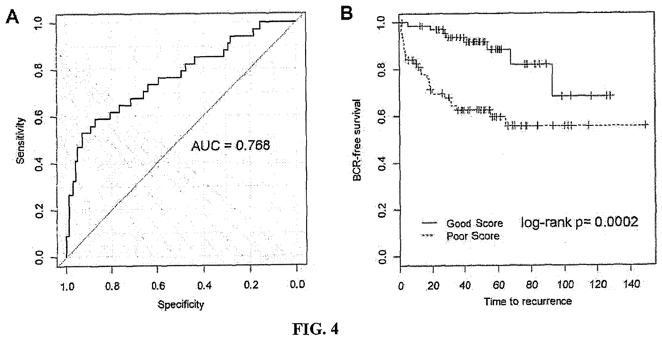

Figure 4 shows experimental data (A) ROC curve using 31 biomarkers from Myriad Genetics (AUC = 0.77). (B) K-M curve of BCR-free survival for samples from Taylor et al. (log-rank test p= 0.0002) using Myriad biomarkers.

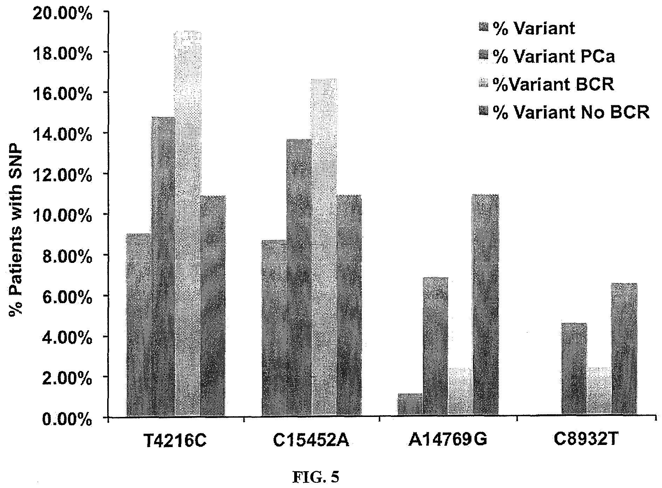

Figure 5 shows data for mitochondrial SNPs identified by RNAseq analysis. %Variant represents variation in the general population as obtained from MitoMap. Additional columns were frequencies observed in all RNAseq cases from this study, those with BCR, and those without BCR.

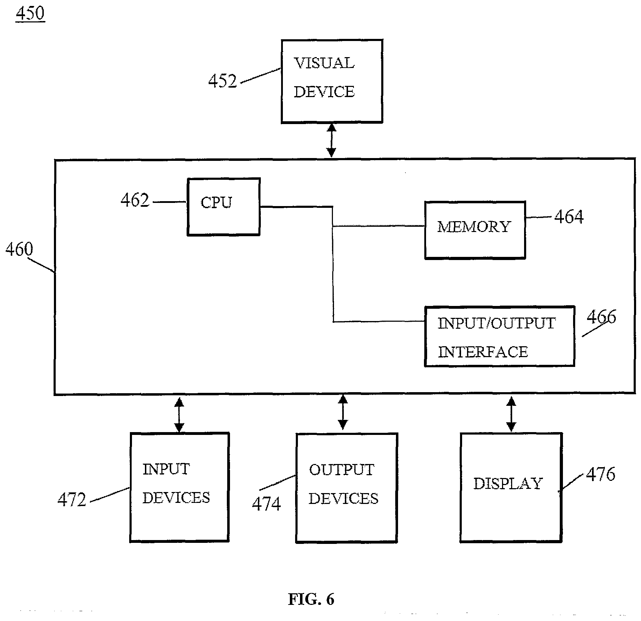

Figure 6 shows a visualization device.

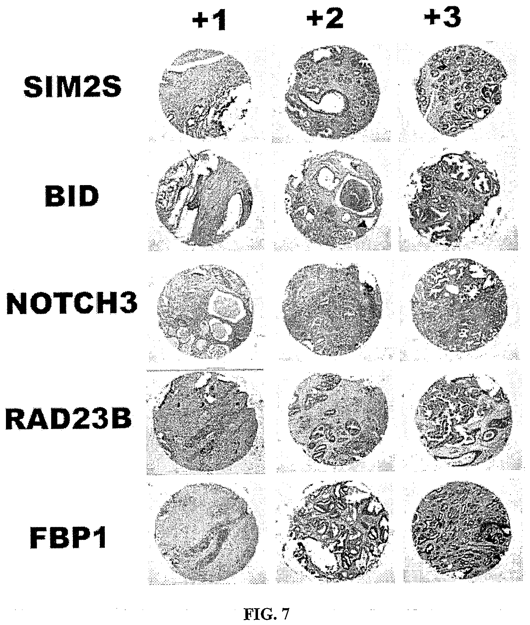

Figure 7 shows representative cores from the stained TMA are indicated for each of the five antibodies tested.

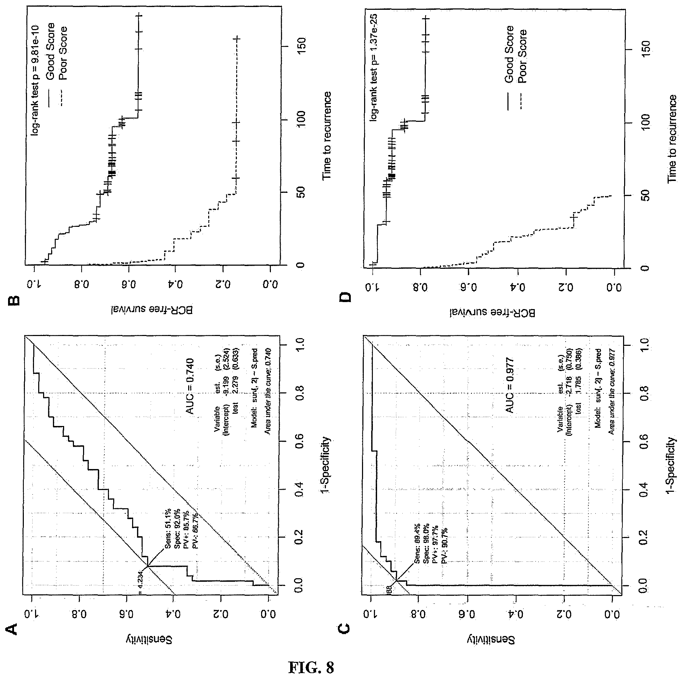

Figure 8 shows data on Receiver-operator characteristic (ROC) and Kaplan-Meier (K-M) survival curves for two groups defined by clinical parameters alone (A-B) or clinical parameters combined with the biomarker genes described in Table 4 (C-D). (A) ROC curve using clinical parameters only (AUC = 0.74). (B) K-M curve of BCR-free survival using clinical parameters only. (C) ROC curve using biomarker score including clinical variables (AUC = 0.97). Sensitivity of 89% and specificity of 98% were obtained. (D) K-M curve of BCR-free survival (log-rank test p=1.37e-25) including clinical variables and stratified by the optimal cutoff (= 2.088) from the ROC curve.

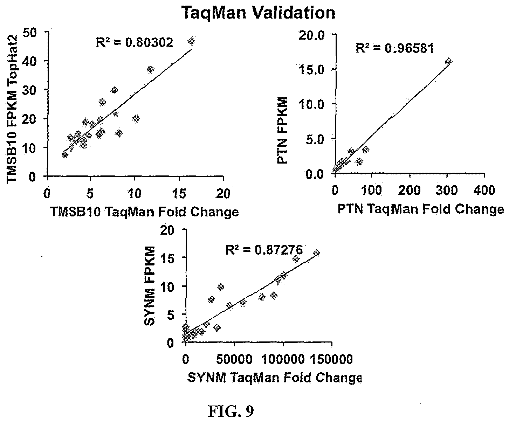

Figure 9 shows data on TaqMan validation of a RNAseq data. TaqMan Fold Change was calculated for three genes (PTN, SYNM, and TMSB10) and plotted against FPKM values.

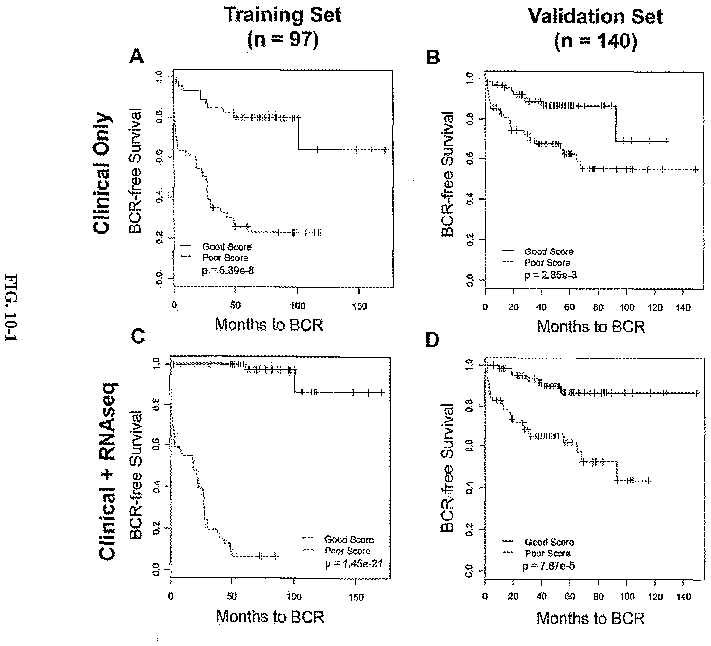

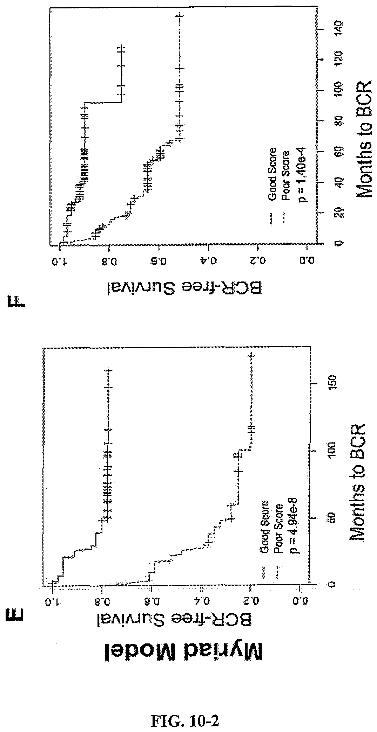

Figure 10 shows Kaplan-Meier survival curves for 97 cases in the training set analyzed by RNAseq (A, C, and E) and for 140 cases in the validation set from Taylor et al. (B, D, and F). K-M curves using clinical parameters alone are shown in panels A and B. K-M curves using clinical parameters combined with the 24 RNA biomarker genes as described in Table 5 are shown in panels C and D. K-M curves using 31 biomarkers from Myriad Genetics are shown in panels E and F.

### DETAILED DESCRIPTION

It is to be understood that the terminology used herein is for the purpose of describing particular aspects only, and is not intended to be limiting, since the scope of the present invention will be limited only by the appended claims.

Unless defined otherwise, all technical and scientific terms used herein have the same meaning as commonly understood by one of ordinary skill in the art to which this disclosure belongs. Although any methods and materials similar or equivalent to those described herein can also be used in the practice or testing of the present disclosure, the preferred methods and materials are now described.

The citation of any publication is for its disclosure prior to the filing date and should not be construed as an admission that the present disclosure is not entitled to antedate such publication by virtue of prior disclosure. Further, the dates of publication provided could be different from the actual publication dates that may need to be independently confirmed.

As will be apparent to those of skill in the art upon reading this disclosure, each of the individual aspects described and illustrated herein has discrete components and features which may be readily separated from or combined with the features of any of the other several aspects without departing from the scope of the present disclosure. Any recited method can be carried out in the order of events recited or in any other order that is logically possible.

Aspects of the present disclosure will employ, unless otherwise indicated, techniques of medicine, organic chemistry, biochemistry, molecular biology, pharmacology, and the like, which are within the skill of the art. Such techniques are explained fully in the literature.

It must be noted that, as used in the specification and the appended claims, the singular forms "a," "an," and "the" include plural referents unless the context clearly dictates otherwise.

In this specification and in the claims that follow, reference will be made to a number of terms that shall be defined to have the following meanings unless a contrary intention is apparent.

Prior to describing the various aspects, the following definitions are provided and should be used unless otherwise indicated.

"Biomarker" means a polynucleotide, polypeptide, or polynucleotide encoding a polypeptide that is differentially present (i.e., increased or decreased) in a biological sample from a subject or a group of subjects having a first phenotype (e.g., having a disease) as compared to a biological sample from a subject or group of subjects having a second phenotype (e.g., not having the disease). A biomarker is preferably differentially present at a level that is statistically significant (i.e., a p-value less than 0.05 and/or a q-value of less than 0.10 as determined using either Welch's T-test or Wilcoxon's rank-sum Test). In certain embodiments, the biomarker may be differentially present at any level, but is generally present at a level that is increased by at least 5%, by at least 10%, by at least 15%, by at least 20%, by at least 25%, by at least 30%, by at least 35%, by at least 40%, by at least 45%, by at least 50%, by at least 55%, by at least 60%, by at least 65%, by at least 70%, by at least 75%, by at least 80%, by at least 85%, by at least 90%, by at least 95%, by at least 100%, by at least 110%, by at least 120%, by at least 130%, by at least 140%, by at least 150%, or more; or is generally present at a level that is decreased by at least 5%, by at least 10%, by at least 15%, by at least 20%, by at least 25%, by at least 30%, by at least 35%, by at least 40%, by at least 45%, by at least 50%, by at least 55%, by at least 60%, by at least 65%, by at least 70%, by at least 75%, by at least 80%, by at least 85%, by at least 90%, by at least 95%, or by 100% (i.e., absent).

The "level" of one or more biomarkers means the absolute or relative amount or concentration of the biomarker in the sample.

"Sample" or "biological sample" means biological material isolated from a subject. The biological sample may contain any biological material suitable for detecting the desired biomarkers, and may comprise cellular and/or non-cellular material from the subject. The sample can be isolated from any suitable biological tissue or fluid such as, for example, prostate tissue, blood, blood plasma, urine, or cerebral spinal fluid (CSF).

"Subject" means any animal, but is preferably a mammal, such as, for example, a human, monkey, mouse, or rabbit.

A "reference level" of a biomarker means a level of the biomarker that is indicative of a particular disease state, phenotype, or lack thereof, as well as combinations of disease states, phenotypes, or lack thereof a "positive" reference level of a biomarker means a level that is indicative of a particular disease state or phenotype. A "negative" reference level of a biomarker means a level that is indicative of a lack of a particular disease state or phenotype. For example, a "prostate cancer-positive reference level" of a biomarker means a level of a biomarker that is indicative of a positive diagnosis of prostate cancer in a subject, and a "prostate cancer-negative reference level" of a biomarker means a level of a biomarker that is indicative of a negative diagnosis of prostate cancer in a subject. A "reference level" of a biomarker may be an absolute or relative amount or concentration of the biomarker, a presence or absence of the biomarker, a range of amount or concentration of the biomarker, a minimum and/or maximum amount or concentration of the biomarker, a mean amount or concentration of the biomarker, and/or a median amount or concentration of the biomarker; and, in addition, "reference levels" of combinations of biomarkers may also be ratios of absolute or relative amounts or concentrations of two or more biomarkers with respect to each other. Appropriate positive and negative reference levels of biomarkers for a particular disease state, phenotype, or lack thereof may be determined by measuring levels of desired biomarkers in one or more appropriate subjects, and such reference levels may be tailored to specific populations of subjects (e.g., a reference level may be age-matched so that comparisons may be made between biomarker levels in samples from subjects of a certain age and reference levels for a particular disease state, phenotype, or lack thereof in a certain age group). Such reference levels may also be tailored to specific techniques that are used to measure levels of biomarkers in biological samples (e.g., Quantitative PCR of mRNA, florescent hybridization probes, antibodies that bind biomarkers etc.), where the levels of biomarkers may differ based on the specific technique that is used.

"Prostate cancer" refers to a disease in which cancer develops in the prostate, a gland in the male reproductive system. "Low grade" or "lower grade" prostate cancer refers to non-metastatic prostate cancer, including malignant tumors with low potential for metastisis (i.e. prostate cancer that is considered to be "less aggressive"). Cancer tumors that are confined to the prostate (i.e. organ-confined, OC) are considered to be less aggressive prostate cancer. "High grade" or "higher grade" prostate cancer refers to prostate cancer that has metastasized in a subject, including malignant tumors with high potential for metastasis (prostate cancer that is considered to be "aggressive"). Cancer tumors that are not confined to the prostate (i.e. non-organ-confined, NOC) are considered to be aggressive prostate cancer. Tumors that are confined to the prostate (i.e., organ confined tumors) are considered to be less aggressive than tumors which are not confined to the prostate (i.e., non-organ confined tumors). "Aggressive" prostate cancer progresses, recurs and/or is the cause of death. Aggressive cancer may be characterized by one or more of the following: non-organ confined (NOC), association with extra capsular extensions (ECE), association with seminal vesicle invasion (SVI), association with lymph node invasion (LN), association with a Gleason Score major or Gleason Score minor of 4, and/or association with a Gleason Score Sum of 8 or higher. In contrast "less aggressive" cancer is confined to the prostate (organ confined, OC) and is not associated with extra capsular extensions (ECE), seminal vesicle invasion (SVI), lymph node invasion (LN), a Gleason Score major or Gleason Score minor of 4, or a Gleason Score Sum of 8 or higher.

### Predicting the Progression of Prostate Cancer in a Subject Diagnosed with Prostate Cancer Based on Biomarker Profiles

The present disclosure relates to methods of predicting the progression of prostate cancer in a subject, comprising analyzing a biological sample from a subject diagnosed with prostate cancer to determine the level(s) of four, five, six, seven or more biomarkers for prostate cancer in the sample, and comparing the level(s) of the biomarkers in the sample to prostate cancer-positive and/or prostate cancer-negative reference levels of the biomarkers, and wherein at least the biomarkers SYNM, IFT57, ITPR1, and PTN are analyzed and compared. Typically, at least eight, nine, ten, or more biomarkers are analyzed and compared. SYMN, SNORA20, HIST1H1C, IFT57, IGFBP3, ITPR1, PTN, EIF2D, and RPL23AP53 and optionally one or more other biomarkers may be analyzed and compared. CDC37L1, COL15A1, COL3A1, EIF2D, HIST1H1C, IFT57, IGFBP3, ITPR1, LBH, MED4, MEMO1, PTN, RPL23AP53, SIRT1, SNORA20, SRSF3, and SYNM and optionally one or more other biomarkers may be analyzed and compared. BTG2, CDC37L1, COL15A1, COL3A1, EIF2D, FDPS, HIST1H1C, HIST1H2BG, IFT57, IGFBP3, ITPR1, LBH, LOC284801, MARCH5, MED4, MEMO1, MXI1, PTN, RPL23AP53, SACM1L, SIRT1, SNORA20, SRSF3, and SYNM may be analyzed and compared. ABCC5, BTG2, CDC37L1, CHRDL1, COL15A1, COL3A1, EIF2D, HIST1H1C, IFT57, IGFBP3, ITPR1, LBH, MARCH5, MED4, MEMO1, MIR663B, NAE1, PTN, RPL23AP53, SIRT1, SNORA20, SRSF3, SYNM, and TAS2R30 may be analyzed and compared. The above biomarkers may be used in any of the methods disclosed herein.

The present disclosure provides methods of predicting the progression of prostate cancer in a subject, comprising analyzing a biological sample from a subject diagnosed with prostate cancer to determine the level(s) of four or more biomarkers for prostate cancer in the sample, where the four or more biomarkers are selected from CDC37L1, COL15A1, COL3A1, EIF2D, HIST1H1C, IFT57, IGFBP3, ITPR1, LBH, MED4, MEMO1, PTN, RPL23AP53, SIRT1, SNORA20, SRSF3, and SYNM; and comparing the level(s) of the four or more biomarkers in the sample to prostate cancer-positive and/or prostate cancer-negative reference levels of the four or more biomarkers in order to determine whether the subject is predisposed to developing a less aggressive or a highly aggressive prostate cancer.

The present disclosure provides methods of predicting the progression of prostate cancer in a subject, comprising analyzing a biological sample from a subject diagnosed with prostate cancer to determine the level(s) of four or more biomarkers for prostate cancer in the sample, where the four or more biomarkers are selected from BTG2, CDC37L1, COL15A1, COL3A1, EIF2D, FDPS, HIST1H1C, HIST1H2BG, IFT57, IGFBP3, ITPR1, LBH, LOC284801, MARCH5, MED4, MEMO1, MXI1, PTN, RPL23AP53, SACM1L, SIRT1, SNORA20, SRSF3, and SYNM; and comparing the level(s) of the four or more biomarkers in the sample to prostate cancer-positive and/or prostate cancer-negative reference levels of the four or more biomarkers in order to determine whether the subject is predisposed to developing a less aggressive or a highly aggressive prostate cancer. As disclosed herein, the biomarkers may be five, six, seven, eight, nine, ten, or more.

As disclosed herein, the biomarkers may be selected from CDC37L1, COL15A1, COL3A1, EIF2D, HIST1H1C, IFT57, IGFBP3, ITPR1, LBH, MED4, MEMO1, PTN, RPL23AP53, SIRT1, SNORA20, SRSF3, and SYNM. In certain aspects, the biomarkers analyzed and compared include CDC37L1, COL15A1, COL3A1, EIF2D, HIST1H1C, IFT57, IGFBP3, ITPR1, LBH, MED4, MEMO1, PTN, RPL23AP53, SIRT1, SNORA20, SRSF3, and SYNM and optionally one or more biomarkers.

The disclosure provides methods of predicting the progression of prostate cancer in a subject, comprising analyzing a biological sample from a subject diagnosed with prostate cancer to determine the level(s) of four or more biomarkers for prostate cancer in the sample, where the four or more biomarkers are selected from ABCC5, BTG2, CDC37L1, CHRDL1, COL15A1, COL3A1, EIF2D, HIST1H1C, IFT57, IGFBP3, ITPR1, LBH, MARCH5, MED4, MEMO1, MIR663B, NAE1, PTN, RPL23AP53, SIRT1, SNORA20, SRSF3, SYNM, and TAS2R30; and comparing the level(s) of the four or more biomarkers in the sample to prostate cancer-positive and/or prostate cancer-negative reference levels of the four or more biomarkers in order to determine whether the subject is predisposed to developing a less aggressive or a highly aggressive prostate cancer. As disclosed herein, the biomarkers may be five, six, seven, eight, nine, ten, or more. As disclosed herein, the biomarkers may be selected from SNORA20, HIST1H1C, IFT57, MIR663B, IGFBP3, ITPR1, PTN, MARCH5, EIF2D, RPL23AP53 or selected from SNORA20, HIST1H1C, IFT57, IGFBP3, ITPR1, PTN, MARCH5, EIF2D, and RPL23AP53. As disclosed herein, the biomarkers analyzed and compared may include SYNM, SNORA20, HIST1H1C, IFT57, MIR663B, IGFBP3, ITPR1, PTN, MARCH5, EIF2D, and RPL23AP53 or comprise SNORA20, HIST1H1C, IFT57, IGFBP3, ITPR1, PTN, MARCH5, EIF2D, and RPL23AP53 and optionally one or more biomarkers.

In certain aspects, the disclosure contemplates methods wherein a subject is already diagnosed with prostate cancer. The subject may have surgery to remove the prostate tissue along with radiation therapy. A sample of the prostate cancer tissue is analyzed for the expression of biomarkers as claimed. The biomarkers will have increased or decreased expression levels when comparing to a typical prostate cancer profile or a less aggressive prostate cancer sample profile, or a highly aggressive prostate cancer sample profile. The larger the changes and number of the biomarker expression profiles are compared to the less aggressive or typical reference levels, the more likely the subject will experience recurrence. Alternatively, the smaller the changes and number of the biomarker expression profiles are to the highly aggressive reference levels, the more likely the subject will experience recurrence. In such a situation, a clinician would suggest an aggressive chemotherapy regiment.

In certain aspects, the disclosure contemplates methods wherein a subject is diagnosed with prostate cancer but has not yet had surgery or radiotherapy. A prostate tissue sample is obtained before surgery and analyzed for the expression of biomarkers as claimed in order to determine whether surgery and/or radiation are warranted. The biomarkers will have increased or decreased expression levels when comparing to a typical prostate cancer or a less aggressive prostate cancer sample reference levels, or a highly aggressive prostate cancer sample reference levels. The smaller the changes and number of the biomarker expression profiles are compared to the less aggressive or typical reference levels, the more likely the subject is advised not to have surgery and/or radiotherapy. In such a situation, a clinician may also suggest a less aggressive chemotherapy regiment. The larger the changes and number of the biomarker expression profiles are compared to the highly aggressive reference levels, the more likely the subject would be advised to undergo surgery and/or radiation treatment and then has radical prostatectomy (RP), external beam radiation therapy, and/or a radioactive seed implant (brachytherapy) followed by an aggressive chemotherapy regiment.

### Diagnosis of Prostate Cancer

The present disclosure relates to the identification of biomarkers for prostate cancer, which allows for the diagnosis of (or for aiding in the diagnosis of) prostate cancer in subjects presenting one or more symptoms of prostate cancer. The present disclosure relates to a method of diagnosing whether a subject has prostate cancer, comprising analyzing a biological sample from a subject to determine the level(s) of four or more biomarkers disclosed herein for prostate cancer in the sample, where the four or more biomarkers are selected from ABCC5, BTG2, CDC37L1, CHRDL1, COL15A1, COL3A1, EIF2D, HIST1H1C, IFT57, IGFBP3, ITPR1, LBH, MARCH5, MED4, MEMO1, MIR663B, NAE1, PTN, RPL23AP53, SIRT1, SNORA20, SRSF3, SYNM, and TAS2R30, and comparing the level(s) of the four or more biomarkers in the sample to prostate cancer-positive and/or prostate cancer-negative reference levels of the four or more biomarkers in order to diagnose whether the subject has prostate cancer. The biomarkers analyzed and compared may include SYNM, IFT57, ITPR1, and PTN, and optionally one or more biomarkers. The biomarkers may be five, six, seven, eight, nine, ten, or more, all, or combinations thereof. The biomarkers may be selected from SNORA20, HIST1H1C, IFT57, MIR663B, IGFBP3, ITPR1, PTN, MARCH5, EIF2D, and RPL23AP53 or selected from SNORA20, HIST1H1C, IFT57, IGFBP3, ITPR1, PTN, MARCH5, EIF2D, and RPL23AP53. The biomarkers analyzed and compared may include SYNM, SNORA20, HIST1H1C, IFT57, MIR663B, IGFBP3, ITPR1, PTN, MARCH5, EIF2D, and RPL23AP53 and optionally one or more biomarkers or include SYNM, SNORA20, HIST1H1C, IFT57, IGFBP3, ITPR1, PTN, MARCH5, EIF2D, and RPL23AP53 and optionally one or more biomarkers.

As disclosed herein, the biomarkers analyzed and compared may include CDC37L1, COL15A1, COL3A1, EIF2D, HIST1H1C, IFT57, IGFBP3, ITPR1, LBH, MED4, MEMO1, PTN, RPL23AP53, SIRT1, SNORA20, SRSF3, and SYNM. The biomarkers analyzed and compared may include BTG2, CDC37L1, COL15A1, COL3A1, EIF2D, FDPS, HIST1H1C, HIST1H2BG, IFT57, IGFBP3, ITPR1, LBH, LOC284801, MARCH5, MED4, MEMO1, MXI1, PTN, RPL23AP53, SACM1L, SIRT1, SNORA20, SRSF3, and SYNM.

The present disclosure relates to a method of diagnosing whether a subject has prostate cancer, comprising analyzing a biological sample from a subject to determine the level(s) of biomarkers for prostate cancer in the sample, where the biomarkers are selected from SYNM, IFT57, ITPR1, and PTN and comparing the level(s) of the biomarkers in the sample to prostate cancer-positive and/or prostate cancer-negative reference levels of the biomarkers in order to diagnose whether the subject has prostate cancer.

The biological sample may be prostate tissue or other bodily fluid such as blood, serum, or urine.

The present disclosure relates to a method of diagnosing whether a subject has prostate cancer, comprising analyzing a biological sample from a subject to determine the presence or absence of one or more gene mutations for prostate cancer in the sample, where the one or more gene mutation are selected from T4216C of ND1, C15452A of Cytb, A14769G of Cytb, and C8932T of ATPase6.

Disclosed herein is a method of diagnosing (or aiding in diagnosing) whether a subject has prostate cancer comprising (1) analyzing a biological sample from a subject to determine the level(s) of one or more biomarkers of prostate cancer in the sample and (2) comparing the level(s) of the one or more biomarkers in the sample to prostate cancer-positive and/or prostate cancer-negative reference levels of the four or more biomarkers in order to diagnose (or aid in the diagnosis of) whether the subject has prostate cancer.

Typically, as described herein, the four or more biomarkers are selected from CDC37L1, COL15A1, COL3A1, EIF2D, HIST1H1C, IFT57, IGFBP3, ITPR1, LBH, MED4, MEMO1, PTN, RPL23AP53, SIRT1, SNORA20, SRSF3, and SYNM or selected from BTG2, CDC37L1, COL15A1, COL3A1, EIF2D, FDPS, HIST1H1C, HIST1H2BG, IFT57, IGFBP3, ITPR1, LBH, LOC284801, MARCH5, MED4, MEMO1, MXI1, PTN, RPL23AP53, SACM1L, SIRT1, SNORA20, SRSF3, and SYNM or selected from ABCC5, BTG2, CDC37L1, CHRDL1, COL15A1, COL3A1, EIF2D, HIST1H1C, IFT57, IGFBP3, ITPR1, LBH, MARCH5, MED4, MEMO1, MIR663B, NAE1, PTN, RPL23AP53, SIRT1, SNORA20, SRSF3, SYNM, and TAS2R30. When such a method is used to aid in the diagnosis of prostate cancer, the results of the method may be used along with other methods (or the results thereof) useful in the clinical determination of whether a subject has prostate cancer.

Any suitable method described herein may be used to analyze the biological sample in order to determine the level(s) of the four or more biomarkers in the sample. Suitable methods include quantitative PCR, florescent probes, chromatography (e.g., HPLC, gas chromatography, liquid chromatography), mass spectrometry (e.g., MS, MS-MS), enzyme-linked immunosorbent assay (ELISA), antibody linkage, other immunochemical techniques, and combinations thereof. Further, the level(s) of the biomarkers may be measured indirectly, for example, by using an assay that measures the level of a compound (or compounds) that correlates with the level of the biomarker(s) that are desired to be measured.

As described herein, the levels of the biomarkers selected from CDC37L1, COL15A1, COL3A1, EIF2D, HIST1H1C, IFT57, IGFBP3, ITPR1, LBH, MED4, MEMO1, PTN, RPL23AP53, SIRT1, SNORA20, SRSF3, and SYNM or selected from BTG2, CDC37L1, COL15A1, COL3A1, EIF2D, FDPS, HIST1H1C, HIST1H2BG, IFT57, IGFBP3, ITPR1, LBH, LOC284801, MARCH5, MED4, MEMO1, MXI1, PTN, RPL23AP53, SACM1L, SIRT1, SNORA20, SRSF3, and SYNM or selected from ABCC5, BTG2, CDC37L1, CHRDL1, COL15A1, COL3A1, EIF2D, HIST1H1C, IFT57, IGFBP3, ITPR1, LBH, MARCH5, MED4, MEMO1, MIR663B, NAE1, PTN, RPL23AP53, SIRT1, SNORA20, SRSF3, SYNM, and TAS2R30, may be determined in the methods of diagnosing and methods of aiding in diagnosing whether a subject has prostate cancer. For example, the level(s) four or more biomarkers, five or more biomarkers, six or more biomarkers, seven or more biomarkers, eight or more biomarkers, nine or more biomarkers, ten or more biomarkers, etc., including a combination of all of the biomarkers and combinations thereof or any fraction thereof, may be determined and used in such methods. Determining levels of combinations of the biomarkers may allow greater sensitivity and specificity in diagnosing prostate cancer and aiding in the diagnosis of prostate cancer, and may allow better differentiation of prostate cancer from other prostate disorders (e.g. benign prostatic hypertrophy (BPH), prostatitis, etc.) or other cancers that may have similar or overlapping biomarkers to prostate cancer (as compared to a subject not having prostate cancer). For example, ratios of the levels of certain biomarkers (and non-biomarker compounds) in biological samples may allow greater sensitivity and specificity in diagnosing prostate cancer and aiding in the diagnosis of prostate cancer and may allow better differentiation of prostate cancer from other cancers or other disorders of the prostate that may have similar or overlapping biomarkers to prostate cancer (as compared to a subject not having prostate cancer).

Biomarkers described herein that are specific for diagnosing prostate cancer (or aiding in diagnosing prostate cancer) in a certain type of sample (e.g., prostate tissue sample, urine sample, or blood plasma sample) may also be used. For example, when the biological sample is prostate tissue, four or more biomarkers selected from CDC37L1, COL15A1, COL3A1, EIF2D, HIST1H1C, IFT57, IGFBP3, ITPR1, LBH, MED4, MEMO1, PTN, RPL23AP53, SIRT1, SNORA20, SRSF3, and SYNM or selected from BTG2, CDC37L1, COL15A1, COL3A1, EIF2D, FDPS, HIST1H1C, HIST1H2BG, IFT57, IGFBP3, ITPR1, LBH, LOC284801, MARCH5, MED4, MEMO1, MXI1, PTN, RPL23AP53, SACM1L, SIRT1, SNORA20, SRSF3, and SYNM or selected from ABCC5, BTG2, CDC37L1, CHRDL1, COL15A1, COL3A1, EIF2D, HIST1H1C, IFT57, IGFBP3, ITPR1, LBH, MARCH5, MED4, MEMO1, MIR663B, NAE1, PTN, RPL23AP53, SIRT1, SNORA20, SRSF3, SYNM, and TAS2R30 may be used to diagnose (or aid in diagnosing) whether a subject has prostate cancer.

As disclosed herein, after the level(s) of the biomarkers in the sample are determined, the level(s) are compared to prostate cancer-positive and/or prostate cancer-negative reference levels to aid in diagnosing or to diagnose whether the subject has prostate cancer. Levels of the biomarkers in a sample matching the prostate cancer-positive reference levels (e.g., levels that are the same as the reference levels, substantially the same as the reference levels, above and/or below the minimum and/or maximum of the reference levels, and/or within the range of the reference levels) are indicative of a diagnosis of prostate cancer in the subject. Levels of the biomarkers in a sample matching the prostate cancer-negative reference levels (e.g., levels that are the same as the reference levels, substantially the same as the reference levels, above and/or below the minimum and/or maximum of the reference levels, and/or within the range of the reference levels) are indicative of a diagnosis of no prostate cancer in the subject. In addition, levels of the biomarkers that are differentially present (especially at a level that is statistically significant) in the sample as compared to prostate cancer-negative reference levels are indicative of a diagnosis of prostate cancer in the subject. Levels of the biomarkers that are differentially present (especially at a level that is statistically significant) in the sample as compared to prostate cancer-positive reference levels are indicative of a diagnosis of no prostate cancer in the subject.

As disclosed herein, the level(s) of the biomarkers may be compared to prostate cancer-positive and/or prostate cancer-negative reference levels using various techniques, including a simple comparison (e.g., a manual comparison) of the level(s) of the biomarkers in the biological sample to prostate cancer-positive and/or prostate cancer-negative reference levels. The level(s) of the biomarkers in the biological sample may also be compared to prostate cancer-positive and/or prostate cancer-negative reference levels using statistical analyses (e.g., t-test, Welch's T-test, Wilcoxon's rank sum test, random forest).

As disclosed herein, the methods of diagnosing (or aiding in diagnosing) whether a subject has prostate cancer may also be conducted specifically to diagnose (or aid in diagnosing) whether a subject has less aggressive prostate cancer and/or high aggressive prostate cancer. Such methods comprise (1) analyzing a biological sample from a subject to determine the level(s) of o biomarkers of less aggressive prostate cancer (and/or high aggressive prostate cancer) in the sample and (2) comparing the level(s) of the biomarkers in the sample to less aggressive prostate cancer-positive and/or less aggressive prostate cancer-negative reference levels (or high aggressive prostate cancer-positive and/or high aggressive prostate cancer-negative reference levels) in order to diagnose (or aid in the diagnosis of) whether the subject has less aggressive prostate cancer (or high aggressive prostate cancer).

### Methods of Determining Predisposition to the Recurrence of Prostate Cancer

The identification of gene mutations for prostate cancer also allows for the determination of whether a subject with prostate cancer is predisposed to the recurrence of prostate cancer. The present disclosure relates to a method comprising (1) analyzing a biological sample from a subject to determine the presence or absence of one or more gene mutations for prostate cancer in the sample, where the one or more gene mutation are selected from T4216C of ND1, C15452A of Cytb, A14769G of Cytb, and C8932T of ATPase6. The level(s) of one or more mutations, two or more mutations, three or more mutations, or all of the mutations or any fraction thereof, may be determined and used in methods of determining whether a subject having prostate cancer is predisposed to recurrence of prostate cancer.

The present invention relates to a method of determining the risk of recurrence of prostate cancer in a subject, wherein the method comprises (1) analyzing a prostatectomy sample from a subject to determine the RNA levels of biomarkers BTG2, CDC37L1, COL15A1, COL3A1, EIF2D, FDPS, HIST1H1C, HIST1H2BG, IFT57, IGFBP3, ITPR1, LBH, LOC284801, MARCH5, MED4, MEMO1, MXI1, PTN, RPL23AP53, SACM1L, SIRT1, SNORA20, SRSF3, and SYNM in the sample and (2) comparing the levels of the four or more biomarkers in the sample to prostate cancer-positive and/or prostate cancer-negative reference levels of the one or more biomarkers. The results of the method may be used along with other methods (or the results thereof) useful in the clinical determination.

After the levels of the biomarkers in the sample are determined, the levels are compared to prostate cancer-positive and/or prostate cancer-negative reference levels in order to predict whether the subject is predisposed to recurrence of prostate cancer. Levels of the biomarkers in a sample matching the prostate cancer-positive reference levels (e.g., levels that are the same as the reference levels, substantially the same as the reference levels, above and/or below the minimum and/or maximum of the reference levels, and/or within the range of the reference levels) are indicative of the subject being predisposed to recurrence of prostate cancer. Levels of the biomarkers in a sample matching the prostate cancer-negative reference levels (e.g., levels that are the same as the reference levels, substantially the same as the reference levels, above and/or below the minimum and/or maximum of the reference levels, and/or within the range of the reference levels) are indicative of the subject not being predisposed to recurrence of prostate cancer. In addition, levels of the biomarkers that are differentially present (especially at a level that is statistically significant) in the sample as compared to prostate cancer-negative reference levels are indicative of the subject being predisposed to recurrence of prostate cancer. Levels of the biomarkers that are differentially present (especially at a level that is statistically significant) in the sample as compared to prostate cancer-positive reference levels are indicative of the subject not being predisposed to recurrence of prostate cancer.

Furthermore, it may be possible to determine reference levels of the biomarkers for assessing different degrees of risk (e.g., low, medium, high) in a subject for recurrence of prostate cancer. Such reference levels could be used for comparison to the levels of the biomarkers in a biological sample from a subject.

As with the methods described above, the level(s) of the biomarkers may be compared to prostate cancer-positive and/or prostate cancer-negative reference levels using various techniques, including a simple comparison, one or more statistical analyses, and combinations thereof.

As disclosed herein, the methods of determining whether a subject having prostate cancer is predisposed to recurrence of prostate cancer may also be conducted specifically to determine whether a subject having prostate cancer is predisposed to the recurrence of less aggressive prostate cancer and/or high aggressive prostate cancer.

As also disclosed herein, methods of determining whether a subject having less aggressive prostate cancer is predisposed to developing high aggressive prostate cancer may be conducted using one or more biomarkers or gene mutations disclosed herein.

### Methods of Monitoring Progression/Regression of Prostate Cancer

The present disclosure relates to the identification of biomarkers for prostate cancer, which also allows for monitoring progression/regression of prostate cancer in a subject.

The present disclosure provides methods of monitoring progression or regression of prostate cancer in a subject comprising analyzing a first biological sample from a subject to determine the level(s) of four or more biomarkers for prostate cancer in the sample, and the first sample is obtained from the subject at a first time point; analyzing a second biological sample from a subject to determine the level(s) of the four or more biomarkers, where the second sample is obtained from the subject at a second time point; and comparing the level(s) of four or more biomarkers in the first sample to the level(s) of the four or more biomarkers in the second sample in order to monitor the progression/regression of prostate cancer in the subject; and wherein at least the biomarkers SYNM, IFT57, ITPR1, and PTN are analyzed and compared.

The present disclosure provides methods of predicting the progression of prostate cancer in a subject, comprising analyzing a biological sample from a subject diagnosed with prostate cancer to determine the level(s) of four or more biomarkers for prostate cancer in the sample, where the four or more biomarkers are selected from ABCC5, BTG2, CDC37L1, CHRDL1, COL15A1, COL3A1, EIF2D, HIST1H1C, IFT57, IGFBP3, ITPR1, LBH, MARCH5, MED4, MEMO1, MIR663B, NAE1, PTN, RPL23AP53, SIRT1, SNORA20, SRSF3, SYNM, and TAS2R30; and comparing the level(s) of the four or more biomarkers in the sample to prostate cancer-positive and/or prostate cancer-negative reference levels of the four or more biomarkers in order to determine whether the subject is predisposed to developing a less aggressive or a highly aggressive prostate cancer. As described herein, the biomarkers analyzed and compared may include SYNM, IFT57, ITPR1, and PTN, and optionally one or more biomarkers. As described herein, the biomarkers may be five, six, seven, eight, nine, ten, or more. As described herein, the biomarkers analyzed and compared may include SNORA20, HIST1H1C, IFT57, MIR663B, IGFBP3, ITPR1, PTN, MARCH5, EIF2D, and RPL23AP53 and optionally one or more biomarkers. As described herein, the biomarkers analyzed and compared may include SYNM, SNORA20, HIST1H1C, IFT57, MIR663B, IGFBP3, ITPR1, PTN, MARCH5, EIF2D, and RPL23AP53 and optionally one or more biomarkers. As described herein, the biomarkers analyzed and compared may include SYNM, SNORA20, HIST1H1C, IFT57, IGFBP3, ITPR1, PTN, MARCH5, EIF2D, and RPL23AP53 and optionally one or more biomarkers.

As disclosed herein, the present disclosure provides a method of monitoring progression/regression of prostate cancer in a subject comprising analyzing a first biological sample from a subject to determine the level(s) of biomarkers disclosed herein, e.g., four or more biomarkers for prostate cancer in the sample, where the four or more biomarkers are selected from CDC37L1, COL15A1, COL3A1, EIF2D, HIST1H1C, IFT57, IGFBP3, ITPR1, LBH, MED4, MEMO1, PTN, RPL23AP53, SIRT1, SNORA20, SRSF3, and SYNM, and the first sample is obtained from the subject at a first time point; analyzing a second biological sample from a subject to determine the level(s) of the four or more biomarkers, where the second sample is obtained from the subject at a second time point; and comparing the level(s) of four or more biomarkers in the first sample to the level(s) of the four or more biomarkers in the second sample in order to monitor the progression/regression of prostate cancer in the subject.

The present disclosure provides a method of monitoring progression/regression of prostate cancer in a subject comprising analyzing a first biological sample from a subject to determine the level(s) of biomarkers disclosed herein, e.g., four or more biomarkers for prostate cancer in the sample, where the four or more biomarkers are selected from BTG2, CDC37L1, COL15A1, COL3A1, EIF2D, FDPS, HIST1H1C, HIST1H2BG, IFT57, IGFBP3, ITPR1, LBH, LOC284801, MARCH5, MED4, MEMO1, MXI1, PTN, RPL23AP53, SACM1L, SIRT1, SNORA20, SRSF3, and SYNM, and the first sample is obtained from the subject at a first time point; analyzing a second biological sample from a subject to determine the level(s) of the four or more biomarkers, where the second sample is obtained from the subject at a second time point; and comparing the level(s) of four or more biomarkers in the first sample to the level(s) of the four or more biomarkers in the second sample in order to monitor the progression/regression of prostate cancer in the subject.

The present disclosure provides a method of monitoring progression/regression of prostate cancer in a subject comprising analyzing a first biological sample from a subject to determine the level(s) of biomarkers disclosed herein, e.g., four or more biomarkers for prostate cancer in the sample, where the four or more biomarkers are selected from ABCC5, BTG2, CDC37L1, CHRDL1, COL15A1, COL3A1, EIF2D, HIST1H1C, IFT57, IGFBP3, ITPR1, LBH, MARCH5, MED4, MEMO1, MIR663B, NAE1, PTN, RPL23AP53, SIRT1, SNORA20, SRSF3, SYNM, and TAS2R30, and the first sample is obtained from the subject at a first time point; analyzing a second biological sample from a subject to determine the level(s) of the four or more biomarkers, where the second sample is obtained from the subject at a second time point; and comparing the level(s) of four or more biomarkers in the first sample to the level(s) of the four or more biomarkers in the second sample in order to monitor the progression/regression of prostate cancer in the subject. As described herein, the biomarkers analyzed and compared may include SYNM, IFT57, ITPR1, and PTN, and optionally one or more biomarkers. As described herein, the biomarkers may be five, six, seven, eight, nine, ten, or more. As described herein, the biomarkers may be selected from SNORA20, HIST1H1C, IFT57, MIR663B, IGFBP3, ITPR1, PTN, MARCH5, EIF2D, and RPL23AP53. As described herein, the biomarkers analyzed and compared may include SYNM, SNORA20, HIST1H1C, IFT57, MIR663B, IGFBP3, ITPR1, PTN, MARCH5, EIF2D, and RPL23AP53; or comprise SNORA20, HIST1H1C, IFT57, IGFBP3, ITPR1, PTN, MARCH5, EIF2D, and RPL23AP53 and optionally one or more biomarkers; or SYNM, SNORA20, HIST1H1C, IFT57, IGFBP3, ITPR1, PTN, MARCH5, EIF2D, and RPL23AP53 and optionally one or more biomarkers.

The present disclosure provides a method of monitoring progression/regression of prostate cancer in a subject comprising analyzing a first biological sample from a subject to determine the level(s) of biomarkers for prostate cancer in the sample, where the biomarkers are SYNM, IFT57, ITPR1, and PTN and the first sample is obtained from the subject at a first time point; analyzing a second biological sample from a subject to determine the level(s) of the biomarkers, where the second sample is obtained from the subject at a second time point; and comparing the level(s) of biomarkers in the first sample to the level(s) of the biomarkers in the second sample in order to monitor the progression/regression of prostate cancer in the subject.

The present disclosure provides a method of monitoring progression/regression of prostate cancer in a subject comprising analyzing a biological sample from a subject to determine the presence or absence of one or more gene mutations for prostate cancer in the sample, where the one or more gene mutation are selected from T4216C of ND1, C15452A of Cytb, A14769G of Cytb, and C8932T of ATPase6.

Described herein is a method of monitoring the progression/regression of prostate cancer in a subject comprising (1) analyzing a first biological sample from a subject to determine the level(s) of four or more biomarkers for prostate cancer selected from the biomarkers selected from CDC37L1, COL15A1, COL3A1, EIF2D, HIST1H1C, IFT57, IGFBP3, ITPR1, LBH, MED4, MEMO1, PTN, RPL23AP53, SIRT1, SNORA20, SRSF3, and SYNM or selected from BTG2, CDC37L1, COL15A1, COL3A1, EIF2D, FDPS, HIST1H1C, HIST1H2BG, IFT57, IGFBP3, ITPR1, LBH, LOC284801, MARCH5, MED4, MEMO1, MXI1, PTN, RPL23AP53, SACM1L, SIRT1, SNORA20, SRSF3, and SYNM or selected from ABCC5, BTG2, CDC37L1, CHRDL1, COL15A1, COL3A1, EIF2D, HIST1H1C, IFT57, IGFBP3, ITPR1, LBH, MARCH5, MED4, MEMO1, MIR663B, NAE1, PTN, RPL23AP53, SIRT1, SNORA20, SRSF3, SYNM, and TAS2R30 the first sample obtained from the subject at a first time point, (2) analyzing a second biological sample from a subject to determine the level(s) of the four or more biomarkers, the second sample obtained from the subject at a second time point, and (3) comparing the level(s) of four or more biomarkers in the first sample to the level(s) of the one or more biomarkers in the second sample in order to monitor the progression/regression of prostate cancer in the subject. The results of the method are indicative of the course of prostate cancer (i.e., progression or regression, if any change) in the subject.

As described herein, the change (if any) in the level(s) of the four or more biomarkers over time may be indicative of progression or regression of prostate cancer in the subject. In order to characterize the course of prostate cancer in the subject, the level(s) of the four or more biomarkers in the first sample, the level(s) of the four or more biomarkers in the second sample, and/or the results of the comparison of the levels of the biomarkers in the first and second samples may be compared to prostate cancer-positive, prostate cancer-negative, less aggressive prostate cancer-positive, less aggressive prostate cancer-negative, high-aggressive prostate cancer-positive, and/or high aggressive prostate cancer-negative reference levels as well as less aggressive prostate cancer-positive reference levels that distinguish over high aggressive prostate cancer and/or high aggressive prostate cancer-positive reference levels that distinguish over low aggressive prostate cancer. If the comparisons indicate that the level(s) of the four or more biomarkers are increasing or decreasing over time (e.g., in the second sample as compared to the first sample) to become more similar to the prostate cancer-positive reference levels (or less similar to the prostate cancer-negative reference levels), to the high aggressive prostate cancer reference levels, or, when the subject initially has less aggressive prostate cancer, to the high aggressive prostate cancer-positive reference levels that distinguish over less aggressive prostate cancer, then the results are indicative of prostate cancer progression. If the comparisons indicate that the level(s) of the four or more biomarkers are increasing or decreasing over time to become more similar to the prostate cancer-negative reference levels (or less similar to the prostate cancer-positive reference levels), or, when the subject initially has high aggressive prostate cancer, to less aggressive prostate cancer reference levels and/or to less aggressive prostate cancer-positive reference levels that distinguish over high aggressive prostate cancer, then the results are indicative of prostate cancer regression.

As with the other methods described herein, the comparisons made in the methods of monitoring progression/regression of prostate cancer in a subject may be carried out using various techniques, including simple comparisons, four or more statistical analyses, and combinations thereof.

As described herein, the results of the method may be used along with other methods (or the results thereof) useful in the clinical monitoring of progression/regression of prostate cancer in a subject.

As described above in connection with methods of diagnosing (or aiding in the diagnosis of) prostate cancer, any suitable method may be used to analyze the biological samples in order to determine the level(s) of the biomarkers in the samples. In addition, the level(s) four or more biomarkers, including a combination of all of the biomarkers selected from CDC37L1, COL15A1, COL3A1, EIF2D, HIST1H1C, IFT57, IGFBP3, ITPR1, LBH, MED4, MEMO1, PTN, RPL23AP53, SIRT1, SNORA20, SRSF3, and SYNM or selected from BTG2, CDC37L1, COL15A1, COL3A1, EIF2D, FDPS, HIST1H1C, HIST1H2BG, IFT57, IGFBP3, ITPR1, LBH, LOC284801, MARCH5, MED4, MEMO1, MXI1, PTN, RPL23AP53, SACM1L, SIRT1, SNORA20, SRSF3, and SYNM or selected from ABCC5, BTG2, CDC37L1, CHRDL1, COL15A1, COL3A1, EIF2D, HIST1H1C, IFT57, IGFBP3, ITPR1, LBH, MARCH5, MED4, MEMO1, MIR663B, NAE1, PTN, RPL23AP53, SIRT1, SNORA20, SRSF3, SYNM, and TAS2R30, or any fraction thereof, may be determined and used in methods of monitoring progression/regression of prostate cancer in a subject.

As described herein, such methods could be conducted to monitor the course of prostate cancer in subjects having prostate cancer or could be used in subjects not having prostate cancer (e.g., subjects suspected of being predisposed to developing prostate cancer) in order to monitor levels of predisposition to prostate cancer.

### Methods of Assessing Efficacy of Compositions for Treating Prostate Cancer

The present disclosure relates to the identification of biomarkers and gene mutations for prostate cancer, which also allows for assessment of the efficacy of a composition for treating prostate cancer as well as the assessment of the relative efficacy of two or more compositions for treating prostate cancer. Such assessments may be used, for example, in efficacy studies as well as in lead selection of compositions for treating prostate cancer.

The present disclosure provides a method of assessing the efficacy of a composition for treating prostate cancer comprising analyzing, from a subject having prostate cancer and currently or previously being treated with a composition, a biological sample to determine the level(s) of one or more biomarkers disclosed herein for prostate cancer and comparing the level(s) of the biomarkers in the sample to (a) levels of the biomarkers in a previously-taken biological sample from the subject, where the previously-taken biological sample was obtained from the subject before being treated with the composition, (b) prostate cancer-positive reference levels of the biomarkers, and/or (c) prostate cancer-negative reference levels of the biomarkers. As described herein, the number of biomarkers may be two, three, four, five, six, seven, eight, nine, ten, or more.

The present disclosure provides a method of assessing the efficacy of a composition for treating prostate cancer comprising analyzing, from a subject having prostate cancer and currently or previously being treated with a composition, a biological sample to determine the presence or absence of one or more gene mutations for prostate cancer in the sample, where the one or more gene mutation are selected from T4216C of ND1, C15452A of Cytb, A14769G of Cytb, and C8932T of ATPase6.

The present disclosure provides a method of assessing the relative efficacy of two or more compositions for treating prostate cancer comprising analyzing, from a first subject having prostate cancer and currently or previously being treated with a first composition, a first biological sample to determine the level(s) of one or more biomarkers disclosed herein; analyzing, from a second subject having prostate cancer and currently or previously being treated with a second composition, a second biological sample to determine the level(s) of the biomarkers; and comparing the level(s) of biomarkers in the first sample to the level(s) of the biomarkers in the second sample in order to assess the relative efficacy of the first and second compositions for treating prostate cancer.

Described herein is a method of assessing the efficacy of a composition for treating prostate cancer comprising (1) analyzing, from a subject having prostate cancer and currently or previously being treated with a composition, a biological sample to determine the level(s) of four or more biomarkers selected from the biomarkers of selected from CDC37L1, COL15A1, COL3A1, EIF2D, HIST1H1C, IFT57, IGFBP3, ITPR1, LBH, MED4, MEMO1, PTN, RPL23AP53, SIRT1, SNORA20, SRSF3, and SYNM or selected from BTG2, CDC37L1, COL15A1, COL3A1, EIF2D, FDPS, HIST1H1C, HIST1H2BG, IFT57, IGFBP3, ITPR1, LBH, LOC284801, MARCH5, MED4, MEMO1, MXI1, PTN, RPL23AP53, SACM1L, SIRT1, SNORA20, SRSF3, and SYNM or selected from ABCC5, BTG2, CDC37L1, CHRDL1, COL15A1, COL3A1, EIF2D, HIST1H1C, IFT57, IGFBP3, ITPR1, LBH, MARCH5, MED4, MEMO1, MIR663B, NAE1, PTN, RPL23AP53, SIRT1, SNORA20, SRSF3, SYNM, and TAS2R30 and (2) comparing the level(s) of the biomarkers in the sample to (a) level(s) of the biomarkers in a previously-taken biological sample from the subject, wherein the previously-taken biological sample was obtained from the subject before being treated with the composition, (b) prostate cancer-positive reference levels (including less aggressive prostate cancer-positive and/or high aggressive prostate cancer-positive reference levels) of the biomarkers, (c) prostate cancer-negative reference levels (including less aggressive prostate cancer-negative and/or high aggressive prostate cancer-negative reference levels) of the biomarkers, (d) less aggressive prostate cancer-positive reference levels that distinguish over high aggressive prostate cancer, and/or (e) high aggressive prostate cancer-positive reference levels that distinguish over less aggressive prostate cancer. The results of the comparison are indicative of the efficacy of the composition for treating prostate cancer.

Thus, in order to characterize the efficacy of the composition for treating prostate cancer as described herein, the level(s) of the biomarkers in the biological sample are compared to (1) prostate cancer-positive reference levels, (2) prostate cancer-negative reference levels, (3) previous levels of the biomarkers in the subject before treatment with the composition, (4) less aggressive prostate cancer-positive reference levels that distinguish over high aggressive prostate cancer, and/or (5) high aggressive prostate cancer-positive reference levels that distinguish over less aggressive prostate cancer.

As described herein, when comparing the level(s) of the biomarkers in the biological sample (from a subject having prostate cancer and currently or previously being treated with a composition) to prostate cancer-positive reference levels and/or prostate cancer-negative reference levels, level(s) in the sample matching the prostate cancer-negative reference levels (e.g., levels that are the same as the reference levels, substantially the same as the reference levels, above and/or below the minimum and/or maximum of the reference levels, and/or within the range of the reference levels) are indicative of the composition having efficacy for treating prostate cancer. Levels of the biomarkers in the sample matching the prostate cancer-positive reference levels (e.g., levels that are the same as the reference levels, substantially the same as the reference levels, above and/or below the minimum and/or maximum of the reference levels, and/or within the range of the reference levels) are indicative of the composition not having efficacy for treating prostate cancer. The comparisons may also indicate degrees of efficacy for treating prostate cancer based on the level(s) of the biomarkers.

As described herein, when comparing the level(s) of the biomarkers in the biological sample (from a subject having high aggressive prostate cancer and currently or previously being treated with a composition) less aggressive prostate cancer-positive reference levels that distinguish over high aggressive prostate cancer and/or high aggressive prostate cancer-positive reference levels that distinguish over less aggressive prostate cancer, level(s) in the sample matching the less aggressive prostate cancer-positive reference levels that distinguish over high aggressive prostate cancer (e.g., levels that are the same as the reference levels, substantially the same as the reference levels, above and/or below the minimum and/or maximum of the reference levels, and/or within the range of the reference levels) are indicative of the composition having efficacy for treating prostate cancer. Levels of the biomarkers in the sample matching the high aggressive prostate cancer-positive reference levels that distinguish over less aggressive prostate cancer (e.g., levels that are the same as the reference levels, substantially the same as the reference levels, above and/or below the minimum and/or maximum of the reference levels, and/or within the range of the reference levels) are indicative of the composition not having efficacy for treating prostate cancer.

As described herein, when the level(s) of the biomarkers in the biological sample (from a subject having prostate cancer and currently or previously being treated with a composition) are compared to level(s) of the biomarkers in a previously-taken biological sample from the subject before treatment with the composition, any changes in the level(s) of the biomarkers are indicative of the efficacy of the composition for treating prostate cancer. That is, if the comparisons indicate that the level(s) of the biomarkers have increased or decreased after treatment with the composition to become more similar to the prostate cancer-negative reference levels (or less similar to the prostate cancer-positive reference levels) or, when the subject initially has high aggressive prostate cancer, the level(s) have increased or decreased to become more similar to less aggressive prostate cancer-positive reference levels that distinguish over high aggressive prostate cancer (or less similar to the high aggressive prostate cancer-positive reference levels that distinguish over low aggressive prostate cancer), then the results are indicative of the composition having efficacy for treating prostate cancer. If the comparisons indicate that the level(s) of the biomarkers have not increased or decreased after treatment with the composition to become more similar to the prostate cancer-negative reference levels (or less similar to the prostate cancer-positive reference levels) or, when the subject initially has high aggressive prostate cancer, the level(s) have not increased or decreased to become more similar to less aggressive prostate cancer-positive reference levels that distinguish over high aggressive prostate cancer (or less similar to the high aggressive prostate cancer-positive reference levels that distinguish over less aggressive prostate cancer), then the results are indicative of the composition not having efficacy for treating prostate cancer. The comparisons may also indicate degrees of efficacy for treating prostate cancer based on the amount of changes observed in the level(s) of the biomarkers after treatment. In order to help characterize such a comparison, the changes in the level(s) of the biomarkers, the level(s) of the biomarkers before treatment, and/or the level(s) of the biomarkers in the subject currently or previously being treated with the composition may be compared to prostate cancer-positive reference levels (including less aggressive and high aggressive prostate cancer-positive reference levels), prostate cancer-negative reference levels (including less aggressive and high aggressive prostate cancer-negative reference levels), less aggressive prostate cancer-positive reference levels that distinguish over high aggressive prostate cancer, and/or high aggressive prostate cancer-positive reference levels that distinguish over less aggressive prostate cancer.

Another method described herein for assessing the efficacy of a composition in treating prostate cancer comprises (1) analyzing a first biological sample from a subject to determine the level(s) of biomarkers, the first sample obtained from the subject at a first time point, (2) administering the composition to the subject, (3) analyzing a second biological sample from a subject to determine the level(s) of the biomarkers, the second sample obtained from the subject at a second time point after administration of the composition, and (4) comparing the level(s) of biomarkers in the first sample to the level(s) of the biomarkers in the second sample in order to assess the efficacy of the composition for treating prostate cancer. As indicated above, if the comparison of the samples indicates that the level(s) of the biomarkers have increased or decreased after administration of the composition to become more similar to the prostate cancer-negative reference levels (or less similar to the prostate cancer-positive reference levels) or, when the subject initially has high aggressive prostate cancer, if the level(s) have increased or decreased to become more similar to less aggressive prostate cancer-positive reference levels that distinguish over high aggressive prostate cancer (or less similar to the high aggressive prostate cancer-positive reference levels that distinguish over less aggressive prostate cancer), then the results are indicative of the composition having efficacy for treating prostate cancer. If the comparisons indicate that the level(s) of the biomarkers have not increased or decreased after treatment with the composition to become more similar to the prostate cancer-negative reference levels (or less similar to the prostate cancer-positive reference levels) or, when the subject initially has high aggressive prostate cancer, the level(s) have not increased or decreased to become more similar to less aggressive prostate cancer-positive reference levels that distinguish over high aggressive prostate cancer (or less similar to the high aggressive prostate cancer-positive reference levels that distinguish over less aggressive prostate cancer), then the results are indicative of the composition not having efficacy for treating prostate cancer. The comparison may also indicate a degree of efficacy for treating prostate cancer based on the amount of changes observed in the level(s) of the biomarkers after administration of the composition as discussed above.

Described herein is a method of assessing the relative efficacy of two or more compositions for treating prostate cancer comprising (1) analyzing, from a first subject having prostate cancer and currently or previously being treated with a first composition, a first biological sample to determine the level(s) of biomarkers (2) analyzing, from a second subject having prostate cancer and currently or previously being treated with a second composition, a second biological sample to determine the level(s) of the biomarkers, and (3) comparing the level(s) of biomarkers in the first sample to the level(s) of the biomarkers in the second sample in order to assess the relative efficacy of the first and second compositions for treating prostate cancer. The results are indicative of the relative efficacy of the two compositions, and the results (or the levels of the biomarkers in the first sample and/or the level(s) of the biomarkers in the second sample) may be compared to prostate cancer-positive reference levels (including less aggressive and high aggressive prostate cancer-positive reference levels), prostate cancer-negative reference levels (including less aggressive and high aggressive prostate cancer-negative reference levels), less aggressive prostate cancer-positive reference levels that distinguish over high aggressive prostate cancer, and/or high aggressive prostate cancer-positive reference levels that distinguish over less aggressive prostate cancer to aid in characterizing the relative efficacy.

Each of the methods of assessing efficacy described herein may be conducted on one or more subjects or one or more groups of subjects (e.g., a first group being treated with a first composition and a second group being treated with a second composition).

### Methods of Screening a Composition for Activity in Modulating Biomarkers Associated with Prostate Cancer

The present disclosure provides a method for screening a composition for activity in modulating four or more biomarkers of prostate cancer, comprising contacting one or more cells with a composition; analyzing at least a portion of the cells or a biological sample associated with the cells to determine the level(s) biomarkers disclosed herein; and comparing the level(s) of the biomarkers with predetermined standard levels for the biomarkers to determine whether the composition modulated the level(s) of the biomarkers.

The present disclosure relates to the identification of biomarkers for prostate cancer, which also allows for the screening of compositions for activity in modulating biomarkers associated with prostate cancer, which may be useful in treating prostate cancer. Methods of screening compositions useful for treatment of prostate cancer comprise assaying test compositions for activity in modulating the levels of four or more biomarkers where the four or more biomarkers are selected from CDC37L1, COL15A1, COL3A1, EIF2D, HIST1H1C, IFT57, IGFBP3, ITPR1, LBH, MED4, MEMO1, PTN, RPL23AP53, SIRT1, SNORA20, SRSF3, and SYNM or selected from BTG2, CDC37L1, COL15A1, COL3A1, EIF2D, FDPS, HIST1H1C, HIST1H2BG, IFT57, IGFBP3, ITPR1, LBH, LOC284801, MARCH5, MED4, MEMO1, MXI1, PTN, RPL23AP53, SACM1L, SIRT1, SNORA20, SRSF3, and SYNM or selected from ABCC5, BTG2, CDC37L1, CHRDL1, COL15A1, COL3A1, EIF2D, HIST1H1C, IFT57, IGFBP3, ITPR1, LBH, MARCH5, MED4, MEMO1, MIR663B, NAE1, PTN, RPL23AP53, SIRT1, SNORA20, SRSF3, SYNM, and TAS2R30. Such screening assays may be conducted in vitro and/or in vivo, and may be in any form known in the art useful for assaying modulation of such biomarkers in the presence of a test composition such as, for example, cell culture assays, organ culture assays, and in vivo assays (e.g., assays involving animal models).

Described herein is a method for screening a composition for activity in modulating one or more biomarkers of prostate cancer comprises (1) contacting one or more cells with a composition, (2) analyzing at least a portion of the one or more cells or a biological sample associated with the cells to determine the level(s) of four or more biomarkers of prostate cancer where the four or more biomarkers are selected from CDC37L1, COL15A1, COL3A1, EIF2D, HIST1H1C, IFT57, IGFBP3, ITPR1, LBH, MED4, MEMO1, PTN, RPL23AP53, SIRT1, SNORA20, SRSF3, and SYNM or selected from BTG2, CDC37L1, COL15A1, COL3A1, EIF2D, FDPS, HIST1H1C, HIST1H2BG, IFT57, IGFBP3, ITPR1, LBH, LOC284801, MARCH5, MED4, MEMO1, MXI1, PTN, RPL23AP53, SACM1L, SIRT1, SNORA20, SRSF3, and SYNM or selected from ABCC5, BTG2, CDC37L1, CHRDL1, COL15A1, COL3A1, EIF2D, HIST1H1C, IFT57, IGFBP3, ITPR1, LBH, MARCH5, MED4, MEMO1, MIR663B, NAE1, PTN, RPL23AP53, SIRT1, SNORA20, SRSF3, SYNM, and TAS2R30; and (3) comparing the level(s) of the biomarkers with predetermined standard levels for the biomarkers to determine whether the composition modulated the level(s) of the biomarkers. As discussed above, the cells may be contacted with the composition in vitro and/or in vivo. The predetermined standard levels for the biomarkers may be the levels of the biomarkers in the one or more cells in the absence of the composition. The predetermined standard levels for the biomarkers may also be the level(s) of the biomarkers in control cells not contacted with the composition.

### Methods of Using the Prostate Cancer Biomarkers for Other Types of Cancer

It is believed that some of the biomarkers for major prostate cancer described herein may also be biomarkers for other types of cancer, including, for example, lung cancer or kidney cancer. Therefore, it is believed that at least some of the prostate cancer biomarkers may be used in the methods described herein for other types of cancer. That is, the methods described herein with respect to prostate cancer may also be used for diagnosing (or aiding in the diagnosis of) any type of cancer, methods of monitoring progression/regression of any type of cancer, methods of assessing efficacy of compositions for treating any type of cancer, methods of screening a composition for activity in modulating biomarkers associated with any type of cancer, methods of identifying potential drug targets for any type of cancer, and methods of treating any type of cancer. Such methods could be conducted as described herein with respect to prostate cancer.

### Methods of Using the Prostate Cancer Biomarkers for Other Prostate Disorders

It is believed that some of the biomarkers for prostate cancer described herein may also be biomarkers for prostate disorders (e.g. prostatitis, benign prostate hypertrophy (BHP)) in general. Therefore, it is believed that at least some of the prostate cancer biomarkers may be used in the methods described herein for prostate disorders in general. That is, the methods described herein with respect to prostate cancer may also be used for diagnosing (or aiding in the diagnosis of) a prostate disorder, methods of monitoring progression/regression of a prostate disorder, methods of assessing efficacy of compositions for treating a prostate disorder, methods of screening a composition for activity in modulating biomarkers associated with a prostate disorder, methods of identifying potential drug targets for prostate disorder, and methods of treating a prostate disorder. Such methods could be conducted as described herein with respect to prostate cancer.

### Arrays and Kits Containing Biomarker Binding Molecules, and Systems for Measuring Biomarkers

This disclosure relates to methods of identifying biomarkers utilizing an analytical platform. Methods for amplification and quantification of RNA and DNA associated with genes are well known. As described herein a solid surface comprises an array of probes that hybridize to nucleic acid associated with the biomarkers, e.g., mRNA that encodes the polypeptide or DNA of a gene encoding mRNA or preRNA or amplified nucleic acid thereof. The solid surface may be placed in contact with a sample from a subject and interactions with a biomarker binding molecule may be detected by analytical techniques, e.g., inducing formation of or extinguishing a fluorescent signal upon the biomarker binding molecule binding to the biomarker.

The disclosure contemplates a solid surface array comprising probes to biomarkers disclosed herein for the purpose of detecting the biomarkers. In certain aspects, the disclosure relates to solid surfaces consisting of an array of nucleic acid probes that hybridize to nucleic acids associated with the genes selected from CDC37L1, COL15A1, COL3A1, EIF2D, HIST1H1C, IFT57, IGFBP3, ITPR1, LBH, MED4, MEMO1, PTN, RPL23AP53, SIRT1, SNORA20, SRSF3, and SYNM or selected from BTG2, CDC37L1, COL15A1, COL3A1, EIF2D, FDPS, HIST1H1C, HIST1H2BG, IFT57, IGFBP3, ITPR1, LBH, LOC284801, MARCH5, MED4, MEMO1, MXI1, PTN, RPL23AP53, SACM1L, SIRT1, , SRSF3, and SYNM or selected from ABCC5, BTG2, CDC37L1, CHRDL1, COL15A1, COL3A1, EIF2D, HIST1H1C, IFT57, IGFBP3, ITPR1, LBH, MARCH5, MED4, MEMO1, MIR663B, NAE1, PTN, RPL23AP53, SIRT1, SNORA20, SRSF3, SYNM, and TAS2R30.

Devices for detection of biomarkers may contain surfaces comprising at least one reagent specific for each biomarker in a biomarker groups disclosed herein, wherein the specific reagent is attached to the surface. For example, a sample from a subject may contain nucleic acids or polypeptides associated with the biomarkers SYNM, IFT57, ITPR1, and PTN, or nucleic acids or polypeptides associated with the biomarkers specific for selected from CDC37L1, COL15A1, COL3A1, EIF2D, HIST1H1C, IFT57, IGFBP3, ITPR1, LBH, MED4, MEMO1, PTN, RPL23AP53, SIRT1, SNORA20, SRSF3, and SYNM or selected from BTG2, CDC37L1, COL15A1, COL3A1, EIF2D, FDPS, HIST1H1C, HIST1H2BG, IFT57, IGFBP3, ITPR1, LBH, LOC284801, MARCH5, MED4, MEMO1, MXI1, PTN, RPL23AP53, SACM1L, SIRT1, SNORA20, SRSF3, and SYNM or selected from ABCC5, BTG2, CDC37L1, CHRDL1, COL15A1, COL3A1, EIF2D, HIST1H1C, IFT57, IGFBP3, IGFBP3, ITPR1, LBH, MARCH5, MED4, MEMO1, MIR663B, NAE1, PTN, RPL23AP53, SIRT1, SNORA20, SRSF3, SYNM, and TAS2R30.

A contemplated device of this disclosure may contain at least one reagent specific for each biomarker that measures sample characteristics. In further examples, provided herein are surfaces wherein said reagent specific for a biomarker is a nucleic acid probe or antibody, or fragment thereof, that is specific for the biomarker.

A biomarker is considered "identified" as being useful for aiding in the diagnosis, diagnosis, stratification, monitoring, and/or prediction of neurological disease when it is significantly different between the subsets of peripheral biological samples tested. Levels of a biomarker are "significantly different" when the probability that the particular biomarker has been identified by chance is less than a predetermined value. The method of calculating such probability will depend on the exact method utilizes to compare the levels between the subsets. As will be understood by those in the art, the predetermined value will vary depending on the number of biomarkers measured per sample and the number of samples utilized. Accordingly, predetermined value may range from as high as 50% to as low as 20, 10, 5, 3, 2, or 1%.

As described herein, the level(s) of biomarker(s) may be measured in a biological sample from an individual. The biomarker level(s) may be measured using any available measurement technology that is capable of specifically determining the level of the biomarker in a biological sample. The measurement may be either quantitative or qualitative, so long as the measurement is capable of indicating whether the level of the biomarker in the sample is above or below the reference value. The disclosure contemplates quantitatively measuring nucleic acids in a sample by techniques such as quantitative PCR.

Although some assay formats will allow testing of samples without prior processing of the sample, it is expected that most samples will be processed prior to testing. The process of comparing a measured value and a reference value can be carried out in any convenient manner appropriate to the type of measured value and reference value for the biomarker at issue. According to the present invention, measuring can be performed using quantitative or qualitative measurement techniques, and the mode of comparing a measured value and a reference value can vary depending on the measurement technology employed. For example, when a qualitative calorimetric assay is used to measure biomarker levels, the levels may be compared by visually comparing the intensity of the colored reaction product, or by comparing data from densitometric or spectrometric measurements of the colored reaction product (e.g., comparing numerical data or graphical data, such as bar charts, derived from the measuring device). However, it is expected that the measured values used in the methods of the invention will most commonly be quantitative values (e.g., quantitative measurements of concentration or quantities of nucleic acids). As with qualitative measurements, the comparison can be made by inspecting the numerical data, by inspecting representations of the data.

According to the present invention, the process of comparing may be manual (such as visual inspection by the practitioner of the method) or it may be automated. For example, an assay device (such as a luminometer for measuring chemiluminescent signals) may include circuitry and software enabling it to compare a measured value with a reference value for a biomarker. Alternately, a separate device (e.g., a digital computer) may be used to compare the measured value(s) and the reference value(s). Automated devices for comparison may include stored reference values for the biomarker(s) being measured, or they may compare the measured value(s) with reference values that are derived from contemporaneously measured reference samples.

In some embodiments, the methods of the disclosure utilize simple or binary comparison between the measured level(s) and the reference level(s) (e.g., the comparison between a measured level and a reference level determines whether the measured level is higher or lower than the reference level). For protein biomarkers, a comparison showing that the measured value for the biomarker is lower than the reference value indicates or suggests a diagnosis. According to the present invention, samples may be measured quantitatively (absolute values) or qualitatively (relative values). The respective biomarker levels for a given assessment may or may not overlap.

The present invention relates to kits comprising nucleic acids configured to bind 24 RNA biomarkers for cancer, wherein the biomarkers are BTG2, CDC37L1, COL15A1, COL3A1, EIF2D, FDPS, HIST1H1C, HIST1H2BG, IFT57, IGFBP3, ITPR1, LBH, LOC284801, MARCH5, MED4, MEMO1, MXI1, PTN, RPL23AP53, SACM1L, SIRT1, SNORA20, SRSF3, and SYNM, and wherein the nucleic acids configured to bind nucleic acids are covalently attached to an array. Typically the kit further comprises one or more or all of the components selected from oligonucleotides or pairs configured to bind for copying predetermined nucleic acid sequences associated with the biomarkers, adaptor oligonucleotides configured to bind to the oligonucleotide, adaptor oligonucleotides configure to bind to nucleic acids attached to an array, ligase, circularization ligase, polymerase, a mix of nucleotides, a biotinylated nucleotide, and a streptavidin bead.

In some embodiments, pairs of nucleic acids, or oligos are configured to bind for copying one of the strands of a nucleic acids associated with biomarkers in a sample (DNA or RNA) - acts as a template to copy the nucleic acids associated with the biomarkers. The paired oligos typically contain a first region that binds the nucleic acids associated with the biomarkers (unique to the biomarker) and a second region that binds an adaptor (typically does not bind the unique biomarker sequence). After binding/hybridizing the pair of oligos to the nucleic acid sequences associated with biomarkers, extension of one end of the oligo primers creates a newly formed oligonucleotide having a copy of the biomarker sequence and one of the oligo pairs. Ligation of the newly formed oligonucleotide to the second oligo pair creates a copy of the biomarker sequence flanked by the pair of oligos, including the second regions of the paired oligos that can bind secondary adaptors. Using the secondary adaptors as PCR primers in PCR amplification, one can insert any nucleic acid sequence of interest into on the ends of the nucleic acids associated with the biomarkers. These modified nucleic acids containing copies of the biomarker sequence can be manipulated, identified, and quantified using known methods.

In certain embodiments, it is contemplated that the kits contain nucleic acids configured to bind for copying the identified biomarkers only, and the kits do not contain not nucleic acids configured to bind for copying other biomarker targets.

The disclosure relates to a system comprising an array comprising zones wherein each zone contains unique nucleic acids configured to bind a nucleic acid associated with a unique biomarkers for prostate cancer, a visual device, and a computing system, wherein the array comprises seven or more zones associates with seven or more biomarkers for prostate cancer, wherein the biomarkers SYNM, IFT57, ITPR1, and PTN are uniquely associate with at least four zones.

In certain embodiments, kits according to the invention include the reagents in the form of an array. The array includes at least two different reagents specific for biomarkers (each reagent specific for a different Biomarker) bound to a substrate in a predetermined pattern (e.g., a grid). Accordingly, the present invention provides arrays comprising nucleic acid probes to 24 RNA biomarkers for cancer, wherein the RNA biomarkers are BTG2, CDC37L1, COL15A1, COL3A1, EIF2D, FDPS, HIST1H1C, HIST1H2BG, IFT57, IGFBP3, ITPR1, LBH, LOC284801, MARCH5, MED4, MEMO1, MXI1, PTN, RPL23AP53, SACM1L, SIRT1, SNORA20, SRSF3, and SYNM.

The instructions relating to the use of the kit for carrying out the disclosure generally describe how the contents of the kit are used to carry out the methods of the invention. Instructions may include information as sample requirements (e.g., form, pre-assay processing, and size), steps necessary to measure the biomarker(s), and interpretation of results.

Instructions supplied in the kits of the invention are typically written instructions on a label or package insert (e.g., a paper sheet included in the kit), but machine-readable instructions (e.g., instructions carried on a magnetic or optical storage disk) are also acceptable. In certain embodiments, machine-readable instructions comprise software for a programmable digital computer for comparing the measured values obtained using the reagents included in the kit.

In some embodiments, the determined gene or protein expression is related to the intensity of a signal generated by a biomarker binding molecule, e.g., nucleic acid probe or antibody that binds a biomarker as claimed. The signal may be outputted from a visual device for recording on a computer readable medium. In some embodiments, the outputting may include displaying, printing, storing, and/or transmitting the determined expression. In some embodiments, the determined expression may be transmitted to another system, server and/or storage device for the printing, displaying and/or storing.

The methods of the disclosure are not limited to the steps described herein. The steps may be individually modified or omitted, as well as additional steps may be added.

Unless stated otherwise as apparent from the following discussion, it will be appreciated that terms such as "detecting," "receiving," "quantifying," "mapping," "generating," "registering," "determining," "obtaining," "processing," "computing," "deriving," "estimating," "calculating" "inferring" or the like may refer to the actions and processes of a computer system, or similar electronic computing device, that manipulates and transforms data represented as physical (e.g., electronic) quantities within the computer system's registers and memories into other data similarly represented as physical quantities within the computer system memories or registers or other such information storage, transmission or display devices. Embodiments of the methods of the invention may be implemented using computer software. If written in a programming language conforming to a recognized standard, sequences of instructions designed to implement the methods may be compiled for execution on a variety of hardware platforms and for interface to a variety of operating systems. In addition, embodiments are not described with reference to any particular programming language. It will be appreciated that a variety of programming languages may be used to implement embodiments of the invention.

Figure 6 shows an example of a system 450 that may be used to quantify expression detected by the sensor according to aspects. The system 450 may include any number of modules that communicate with other through electrical or data connections. The modules may be connected via a wired network, wireless network, or combination thereof. The networks may be encrypted. The wired network may be, but is not limited to, a local area network, such as Ethernet, or wide area network. The wireless network may be, but is not limited to, any one of a wireless wide area network, a wireless local area network, a Bluetooth network, a radio frequency network, or another similarly functioning wireless network.

Although the modules of the system are shown as being directly connected, the modules may be indirectly connected to one or more of the other modules of the system. A module may be only directly connected to one or more of the other modules of the system.

It is also to be understood that the system disclosed herein may omit any of the modules illustrated and/or may include additional modules not shown. It is also be understood that more than one module may be part of the system although one of each module is illustrated in the system. It is further to be understood that each of the plurality of modules may be different or may be the same. It is also to be understood that the modules may omit any of the components illustrated and/or may include additional component(s) not shown.

The modules provided within the system may be time synchronized. The system may be time synchronized with other systems, such as those systems that may be on the medical and/or research facility network.

The system 450 may optionally include a visual device 452. The visual device 452 may be any visual device configured to capture changes in a shape, light, or fluorescence. For example, the visual device may include but is not limited to a camera and/or a video recorder. The visual device may be a part of a microscope system. The system 450 may communicate with other visual device(s) and/or data storage device.

The visual device 452 may include a computer system to carry out the image processing. The computer system may further be used to control the operation of the system or a separate system may be included.

The system 450 may include a computing system 460 capable of quantifying the expression. The computing system 460 may be a separate device. The computing system 460 may be a part (e.g., stored on the memory) of other modules, for example, the visual device 452, and controlled by its respective CPUs.

The system 460 may be a computing system, such as a workstation, computer, or the like. The system 460 may include one or more processors (CPU) 462. The processor 462 may be one or more of any central processing units, including but not limited to a processor, or a microprocessor. The processor 462 may be coupled directly or indirectly to one or more computer-readable storage medium (e.g., physical memory) 464. The memory 464 may include one or more memory elements, such random access memory (RAM), read only memory (ROM), disk drive, tape drive, etc., or a combinations thereof. The memory 464 may also include a frame buffer for storing image data arrays. The memory 464 may be encoded or embedded with computer-readable instructions, which, when executed by one or more processors 462 cause the system 460 to carry out various functions.

The system 460 may include an input/output interface 468 configured for receiving information from one or more input devices 472 (e.g., a keyboard, a mouse, joystick, touch activated screen, etc.) and/or conveying information to one or more output devices 474 (e.g., a printing device, a CD writer, a DVD writer, portable flash memory, display 476 etc.). In addition, various other peripheral devices may be connected to the computer platform such as other I/O (input/output) devices.

In some embodiments, the claimed methods may be implemented using software applications that are stored in a memory and executed by a processor (e.g., CPU) provided on the system. In some embodiments, the claimed methods may be implemented using software applications that are stored in memories and executed by CPUs distributed across the system. As such, the modules of the system may be a general purpose computer system that becomes a specific purpose computer system when executing the routine of the disclosure. The modules of the system may also include an operating system and micro instruction code. The various processes and functions described herein may either be part of the micro instruction code or part of the application program or routine (or combination thereof) that is executed via the operating system.

It is to be understood that the embodiments of the invention may be implemented in various forms of hardware, software, firmware, special purpose processes, or a combination thereof. In one embodiment, the invention may be implemented in software as an application program tangible embodied on a computer readable program storage device. The application program may be uploaded to, and executed by, a machine comprising any suitable architecture. The method of the invention may be implemented in the form of a software application running on a computer system, for example, a mainframe, personal computer (PC), handheld computer, server, etc. The software application may be stored on a recording media locally accessible by the computer system and accessible via a hard wired or wireless connection to a network, for example, a local area network, or the Internet.

It is to be further understood that, because some of the constituent system components and method steps depicted in the accompanying figures may be implemented in software, the actual connections between the systems components (or the process steps) may differ depending upon the manner in which the disclosure is programmed. Given the teachings of the disclosure provided herein, one of ordinary skill in the related art will be able to contemplate these and similar implementations or configurations of the disclosure.

### EXPERIMENTS

### Reference Example 1

A panel of ten protein-coding genes and two miRNA genes (RAD23B, FBP1, TNFRSF1A, CCNG2, NOTCH3, ETV1, BID, SIM2, ANXA1, miR-519d, and miR-647) was reported, Long *et al.,* that could be used to separate patients with and without biochemical recurrence (p < 0.001), as well as for the subset of 42 Gleason score 7 patients (p < 0.001). See Am J Pathol, (2011), 179, 46-54. Importantly, these biomarkers could significantly predict clinical recurrence for Gleason score 7 patients. This previous study was limited by the fact that it started from a set of approximately 500 candidate genes and used the DASL technology for quantitation of RNA species. In this study, we have used the genome-wide approach of next-generation RNA sequencing of formalin-fixed paraffin-embedded (FFPE) tissues to identify biomarkers of biochemical recurrence (BCR) following prostatectomy.

Since RNA from FFPE samples is typically fragmented, conventional methods for sequencing library preparation that utilize poly-dT hybridization to capture mRNA will not work. We tested two alternative library preparation methods using the Ribominus kit (Ambion) to remove ribosomal RNA, and the Ovation WT Amplification kit for FFPE samples. Pilot RNA-seq analysis on eleven samples (six from Ribominus, and five from Ovation) determined that the more cost effective option (Ribominus) gives equal or superior sequencing read coverage.

Cancer and benign tissue in slides and formalin-fixed paraffin embedded (FFPE) blocks were identified for regions of 100 cases. These samples were then submitted for processing to obtain 1 mm tissue cores. RNA from 99 samples were prepared of which 79 passed our RNA quality control (QC) analysis for genomic profiling. Samples were also obtained from the Sunnybrook Health Science Center (Toronto, ON) and from the Moffitt Comprehensive Cancer Center (MCCC) in Tampa, FL.

Those who were included met specific inclusion criteria, had available tissue specimens, documented long term follow-up and consented to participate or were included by IRB waiver. The cases were assigned prostate ID numbers to protect their identities. These patients did not receive neo-adjuvant or concomitant hormonal therapy. Their demographic, treatment and long-term clinical outcome data have been collected and recorded in an electronic database. Clinical data recorded include PSA measurements, radiological studies and findings, clinical findings, tissue biopsies and additional therapies that the subjects may have received. In total, 91 samples were sequenced by next generation sequencing: 61 from the VAMC, 23 from Toronto, and 7 from MCCC. Of these cases 43 had biochemical recurrence (BCR) and 48 had no BCR.

Tissue cores (1 mm) were used for RNA preparation rather than sections because of the heterogeneity of samples and the opportunity for obtaining cores with very high percentage tumor content, except for the seven samples from MCCC. MCCC supplied total FFPE RNA that was prepared from five micron unstained sections. H&E stained slides were reviewed by a board certified urologic pathologist to identify regions of cancer to select corresponding areas for cutting of cores from paraffin blocks. Total RNA was prepared at the Winship Cancer Genomics Shared Resource from FFPE cores, using the Omega Biotek FFPE RNA methodology in 96-well format on a MagMax 96 Liquid Handler Robot (Life Technologies, Carlsbad, CA). FFPE RNA was quantitated using a Nanodrop spectrophotometer (Wilmington, DE), and tested for RNA integrity and quality by Taqman analysis of the RPL13a ribosomal protein on a HT7900 real-time PCR instrument (Applied Biosystems, Foster City, CA). Samples with sufficient yield (>500 ng), A₂₆₀/A₂₈₀ ratio > 1.8 and RPL13a C_{T} values less than 30 cycles were used for preparation of RNAseq libraries.

RNA sequencing libraries were prepared using the TruSeq kit (Illumina, Inc) with the following modification. Instead of purifying poly-A RNA using poly-dT primer beads, we removed ribosomal RNA using the Ribominus kit (Ambion). All other steps were performed according to the manufacturer's protocols. RNAseq libraries were analyzed for QC and average size of inserts were approximately 200-300 bp. Samples were multiplexed into three samples per lane on the Illumina Version 3 Flowcells on the HiSeq2000 platform. Samples were sequenced at the Emory GRA Genomics Core Facility and at the Southern California Genotyping Consortium (SCGC). All samples were subjected to 50bp paired-end sequencing.

FASTQ files generated from the Illumina HiSeq2000 were mapped to the human genome using TopHat software, and Cufflinks software was used to generate FPKM values. Genes were filtered to determine if they were detected (defined as FPKM > 1) in each sample. Genes that were detected in 80% of BCR samples or 80% of non-BCR samples were retained, leaving a set of 4432 genes for further analysis.

A prediction model was built using clinical variables, namely, tgleason, prepsa, pstg, and age. Specifically, 1) fit a Cox Proportional Hazards (PH) model using the set of 4 clinical variables, and the resultant coefficients; 2) calculate predictive scores using the coefficients and the set of clinical variables; and 3) divide subjects into higher and lower risk groups based on the median predictive score and perform log rank test to compare BCR between the two groups.

Construct a prediction model using the set of 4432 gene markers for which 80% subjects have an FPKM>1 (i.e., detected expression) in at least one of the BCR group and no BCR group and using the stability selection procedure. Specifically, 1) calculate standardized FPKM values for each gene (with mean of 0 and standard deviation of 1); 2) apply the stability selection approach in combination with randomized lasso PH models for time to BCR to the set of 4432 genes, and obtain a set of selected genes and their estimated coefficients; 3) calculate predictive scores using the coefficients and standardized FPKM values; 4) calculate predictive score with clinical variables by fitting a Cox PH model using the gene predictive scores and the set of clinical variables; and 5) subjects were divided into high and low risk groups based on the median predictive score and perform log rank test to compare BCR between the two risk groups with or without use of clinical variables.

**Table 1 Selected genes and estimated coefficients**

| Selected Genes | Coefficient (Proportion of being selected) | |
|---|---|---|
| SYNM | -0.567 (0.413) | Synemin, Intermediate Filament Protein |
| GPX1 | 0.64 (0.604) | Glutathione Peroxidase 1 |
| TMSB10 | 0.526 (0.446) | Thymosin Beta 10 |
| IFT57 | 0.305 (0.512) | Intraflagellar Transport 57 Homolog (HIPPI) |
| ITPR1 | -0.449 (0.494) | Inositol 1,4,5-Triphosphate Receptor, Type 1 |
| PTN | -1.049 (0.517) | Pleiotrophin |
| LAPTM5 | 0.48 (0.416) | Lysosomal Multispanning Membrane Protein 5 |

### Example 2

A panel of biomarkers has been identified that is able to predict recurrence following radical prostatectomy. Validation studies on separate groups of patients will speed translation of these biomarkers into a clinical lab test that may be translated to widespread clinical application that would give physicians an idea as to what is the best course of treatment for patients with prostate cancer and help avoid unnecessary treatments. Future studies that apply these biomarkers to biopsy or biofluid samples from patients who undergo radiation or active surveillance may be used determine whether they are also useful and discriminating aggressive from indolent disease. In the long run, this will result in better patient outcomes and reduced healthcare costs and treatment side effects.

Through RNAseq analysis of 100 prostatectomy cases, a set of 24 biomarker genes (22 protein coding and two small RNA) were identified to be highly predictive of biochemical recurrence in prostate cancer patients. Several genes were also identified to be differentially expressed in AA patients including ETV5. Furthermore inherited missense mitochondrial SNPs were identified that may predispose patients to BCR.

The comparison of Gleason 3+4 to 4+3 cases identified several interesting genes indicating that increasing miR10A, Twist, HOXC6, and AR expression and decreasing SOX9, WIF1, and WNT5A are associated with increasing tumor grade. Moreover, the fact that the most significant biological annotation was abnormal bone morphology suggests that higher Gleason primary pattern tumors intrinsically express genes that may facilitate metastasis to the bone.

Global RNA-sequencing was performed on 106 formalin-fixed, paraffin-embedded (FFPE) prostatectomy samples from 100 patients at three independent sites. A set of biomarkers of biochemical recurrence was identified composed of a 24-gene panel including 22 protein-coding genes and two non-coding genes. Excellent correlations between TaqMan and RNAseq values, as well as for RNAseq between replicate libraries, was observed. This 24-gene panel was validated on an independent publicly available dataset of 140 patients and outperformed previously published markers based on cell proliferation gene sets. In addition, genes were identified that are differentially expressed between African-American and Caucasian prostate cancer patients, and mitochondrial SNPs that are associated with both race and outcome. Since these biomarkers have been developed on FFPE RNA samples, they may be suitable for rapid clinical translation for prediction of outcome following surgery.

RNA was prepared from FFPE 1mm cores, and followed by QC analysis. A total of 106 RNAseq libraries from 100 patients was prepared: 61 from the AVAMC, 35 libraries from 29 patients from Toronto, and 10 from MCCC. Of these cases 49 had biochemical recurrence (BCR) and 51 had no BCR.

RNAseq analysis of FFPE samples was done using the Ribominus kit (Ambion) to remove ribosomal RNA, followed by library preparation using the Illumina TruSeq kit. Possibly due to the fact that we did not perform a poly-A selection step, a significant number of reads that mapped to gene introns was observed, likely from partially processed mRNAs. Large numbers of reads mapping to intergenic regions was not observe indicating that there was minimal DNA contamination in our samples. Moreover, the level of intronic reads was similar to that observed in RNAseq data derived from fresh frozen samples analyzed by The Cancer Genome Atlas project (TCGA). To validate and verify the accuracy of our RNAseq data, TaqMan analyses was performed on a few select genes and observed excellent correlation of TaqMan and RNAseq data (r² = 0.80-0.97).

RNA was also prepared from separate cores from the same six patients, and prepared separate sequencing libraries on different days. Analysis of the fragment per kilobase of transcript per million mapped reads (FPKM) values from these replicate sequence analyses indicated very strong correlation (r² = 0.70-0.96) for the 5265 genes that were robustly detected in at least 80% of samples and used in our biomarker analyses. The pair of samples with the lowest correlation (UTPC034) had the greatest difference in number of mapped reads (18M vs. 112M), while the paired samples with the highest correlation (UTPC004) both had very deep coverage (94M and 101M mapped reads each). Differential gene expression analysis using both DESeq and EdgeR indicated very few differentially expressed genes between replicate sequencing libraries (14 genes on average). For biomarker analysis, in each case, the library with the higher number of mapped reads was used.

FASTQ files generated from the Illumina HiSeq2000 were mapped to the human genome using TopHat software (version 2.0.8) and Bowtie (version 2.1.0), and Cufflinks software was used to generate FPKM values. Genes were filtered to determine if they were detected (defined as FPKM > 1) in each sample. Genes that were detected in 80% of BCR samples or 80% of non-BCR samples were retained, leaving a set of 5265 protein-coding or non-coding genes for further analysis. Duplicates from six patient samples were removed, and three patients had incomplete clinical data on PSA and stage, leaving 97 samples for biomarker analysis.

Using the set of 5265 genes using 97 samples, a 24-gene prediction model (22 protein-coding and two non-coding) was built using a pre-selection step and a lasso Cox PH model and the final prediction model was built to include the predictive score based on this panel of 24 markers as well as the relevant clinical biomarkers including T-stage, PSA, Gleason score, and age. For comparison, a prediction model was also built using only clinical information, namely, T-stage, PSA, Gleason score, and age, through fitting a Cox PH model. Log-rank test were then performed to compare BCR between the low risk (good score) and high risk (poor score) groups with or without use of clinical variables. Kaplan-Meier analysis (Figure 2d) demonstrated that these markers could significantly discriminate patients at higher and lower risk of recurrence by the log-rank test (p = 3.70e-23) in our training data, more significant than using clinical variables alone (p=0.0003). Prediction of recurrence in terms of the area under the ROC curve (AUC) was greatly improved using our biomarkers in combination with clinical parameters relative to using clinical parameters alone, increasing from 0.75 to 0.99 (Figure 2a and 2c).

**Table 2: Summary of sequencing statistics from RNAseq analysis of 106 FFPE RNA samples**

| Selected Genes | Coefficient | |
|---|---|---|
| ABCC5 | 0.055365 | ATP-Binding Cassette, sub-family C (CFTR/MRP), Member 5 |
| BTG2 | -0.10177 | B-cell Translocation Gene 2 |
| CDC37L1 | -0.16767 | Cell Division Cycle 37-Like 1 |
| CHRDL1 | -0.02268 | Chordin-Like 1 |
| COL15A1 | 0.031294 | Collagen, Type XV, Alpha 1 |
| COL3A1 | 0.182489 | Collagen, Type III, Alpha 1 |
| EIF2D | 0.328023 | Eukaryotic Translation Initiation Factor 2D |
| HIST1H1C | 0.176363 | Histone Cluster 1, H1c |
| IFT57 | 0.246541 | Intraflagellar Transport 57 Homolog |
| IGFBP3 | 0.049884 | Insulin-like Growth Factor Binding Protein 3 |
| ITPR1 | -0.14729 | Inositol 1,4,5-Trisphosphate Receptor, Type 1 |
| LBH | 0.078994 | Limb Bud and Heart Development |
| MARCH5 | -0.22986 | Membrane-associated Ring Finger (C3HC4) 5 |
| MED4 | -0.08749 | Mediator Complex Subunit 4 |
| MEMO1 | 0.095666 | Mediator of Cell Motility 1 |
| MIR663B | -0.36145 | microRNA 663b |
| NAE1 | -0.00864 | NEDD8 Activating Enzyme E1 Subunit 1 |
| PTN | -0.16516 | Pleiotrophin |
| RPL23AP53 | -0.11149 | Ribosomal Protein L23a Pseudogene 53 |
| SIRT1 | -0.1136 | Sirtuin 1 |
| SNORA20 | -0.04845 | Small Nucleolar RNA, H/ACA Box 20 |
| SRSF3 | -0.0684 | Serine/Arginine-rich Splicing Factor 3 |
| SYNM | -0.1004 | Synemin, Intermediate Filament Protein |
| TAS2R30 | -0.00126 | Taste Receptor, Type 2, Member 30 |

To validate this panel of biomarkers, an independent gene expression microarray study was identified with data from 140 prostate cancer patients. Taylor et al., Cancer Cell 18, 11-22 (2010). Using the data from Taylor et al., the final prediction models obtained from the training phase were evaluated. The prediction model was applied based on clinical variables alone showing significant discriminative performance in the validation data as well (p =0.0154, Figure 3b). Since some markers including miRNA markers from our RNAseq analysis are not available in the independent testing data from Taylor et al., the training step was repeated using only markers that are available in the testing data and a second prediction model wsa constructed for the purpose of testing. A second panel of 24 gene markers was identified which had a substantial overlap with the first panel (Table 2). Each prediction model from the training phase was used to generate a predictive score for each subject in the testing data set, and subjects were subsequently divided into high and low risk groups using the median predictive score. Kaplan Meier analysis was performed to compare time to BCR between high (poor score) and low (good score) risk groups, and the biomarkers were very significantly prognostic in this independent validation set (log-rank test p = 6.92e-05, Figure 3d). In addition, ROC analysis indicated an increase of AUC from 0.701 to 0.777 when using these biomarkers in combination with clinical parameters. However, this analysis was limited by the fact that the dataset in Taylor et al. did not include expression for one of our biomarker genes, miR-663b, and thus this represents the lower bound of the ability of these biomarkers to predict outcome.

### Comparison with existing biomarkers

In addition, a direct comparison of the 24 biomarker genes in table two was performed with a set of 31 cell cycle progression genes developed by Myriad Genetics. Cuzick, J. et al. Lancet Oncol 12, 245-55 (2011) One variable, margin, in the model from Cuzick et al. is not available in the independent testing data, so the prediction scores were calcluated from this model after removing the term for margin. Our biomarker genes outperformed the 31 cell cycle progression genes using the data from Taylor et al., which had less significant Kaplan-Meier (log-rank test p = 0.0002) and ROC analyses (AUC = 0.768) (Figure 4).

### Mitochondrial SNP and INDEL analysis

The depth of coverage obtained for mitochondrial RNA sequences was high enough (much greater than 100x) that recurrent single nucleotide polymorphisms (SNPs) in the mtDNA of the patient samples were identify. Analysis results indicated that no recurrent INDELs were found in coding regions of any gene. Only three regions, the DNA replication primer region (bp 302), a hypervariable control region 3 (bp 513), and a 16S ribosomal RNA (bp 2940) contained indels in multiple samples. In addition, a total of 435 SNPs were identified in two or more prostate cancer patients, of which 21 were more frequent in our prostate cancer cohort of 100 patients than in the general population. Four of these SNPs (T4216C, C15452A, A14769G, and C8932T) were of particular interest (Table 3 and Figure 5).

**Table 3: Mitochondrial SNPs of increased frequency in prostate cancer patients**

| **SNP** | **Gene** | **Codon** | **Amino Change** | **% Variant** | **% Variant PCa** | **% Variant BCR** | **% Variant No BCR** |
|---|---|---|---|---|---|---|---|
| T4216C | ND1 | 304 | Tyr -> His | 9.02% | 14.77% | 19.05% | 10.87% |
| C15452A | Cytb | 236 | Leu -> Ile | 8.69% | 13.64% | 16.67% | 10.87% |
| A14769G | Cytb | 8 | Asn -> Ser | 1.11% | 6.82% | 2.38% | 10.87% |
| C8932T | ATPase6 | 136 | Pro -> Ser | 0.07% | 4.55% | 2.38% | 6.52% |

All of these SNPs result in non-synonymous amino-acid substitutions of mitochondrial protein-coding genes. The T4216C and C15452A SNPs almost always co-occur (p-value = 1.57e-13), and are mutually exclusive of the strongly co-occuring A14769G and C8932T SNPs. Moreover, the T4216C/C15452A are not only more frequent in prostate cancer patients, but also are more frequent in patients with BCR, and were not observed in any AA patients. The A14769G/C8932T SNPs were more frequent in patients without BCR and were observed only in AA patients. The number of variant reads (range 82 to 654) relative to consensus reads (range 1-4) indicates that these are likely fixed and inherited alleles, and not due to somatic mutation or heteroplasmy.

### Gleason score analysis

The grading system for prostate cancer is unique in that the final pathological grade is a Gleason sum obtained by assigning a single Gleason grade to the most prevalent pattern, known as the primary patterns and then adding this to another single Gleason grade assigned to the next most prevalent pattern, known as the secondary pattern, to obtain a sum known as the Gleason Score. It has been suggested that primary Gleason 4 pattern and Gleason 3 pattern tumors represent different disease states, and several studies have supported the concept that the primary Gleason pattern of Gleason seven patients is predictive of outcome. To investigate the differences in gene expression, DEseq differential gene expression analysis was performed comparing 43 samples with Gleason 3+4 (primary pattern 3) to 22 samples with Gleason 4+3 (primary pattern 4). Genes (304) were identified differentially expressed between these two patient groups. There were several genes differentially expressed relevant to prostate cancer, including upregulated (miR10A, Twist, HOXC6, AR) and downregulated (ERG, EGF, SOX9, WIF1, WNT5A, SHH) genes in Gleason 4+3 compared to 3+4 cases. IPA pathway analysis also determined that the top biological functions associated with the 304 differentially expressed genes were abnormal bone morphology (p = 7.72e-8), cell differentiation (p = 4.05e-7), genital tumors (p = 1.66e-6), and prostatic bud formation (p = 7.48e-6).

### Example 3

During the course of preparation of 1mm tissue cores for RNA extraction of prostatectomy samples from the Atlanta VA Medical Center (VAMC), a tissue microarray (TMA) was generated that includes cores from 97 VAMC cases with known outcome Since five of our candidate biomarkers (RAD23B, SIM2s, Notch3, BID and FBP1) are upregulated at the RNA level in cases with recurrence and have available commercial antibodes, antibodies to these proteins were tested on our TMAs in order to determine whether the protein level expression was similarly elevated. Five TMA sections were stained with antibodies to RAD23B, SIM2S, Notch3, BID, and FBP1. Intensity of the various immunohistochemical stains were scored blindly as follows; 0-negative, 1+ (weak), 2+ (intermediate) and 3+ (strong). Representative cores are shown in Figure 7.

The expression of these markers was correlated with biochemical recurrence (BCR) using Fisher's exact test with samples divided into categories 0-2+ vs. 3+ for staining and positive vs. negative for BCR. BID (p = 0.0005), SIM2s (p = 0.007), RAD23B (p = 0.05), and FBP1 (p = 0.04) were significantly associated with BCR by Fisher's exact test. NOTCH3 (p = 0.56), did not demonstrate statistical significant association with BCR. We also evaluated the staining in survival analysis by Cox Proportional Hazards model and Log Rank test. Moreover, there was no statistically significant correlation between any of the markers and pathologic stage, Gleason score, patient race, or pre-operative PSA levels. BID, FBP1, RAD23B, and SIM2s may be useful immunohistochemical biomarkers in the prediction of BCR in patients following radical prostatectomy, irrespective of pathologic stage, Gleason score, patient race, or pre-operative PSA levels.

### Example 4

RNA sequencing libraries were prepared on 106 prostatectomy RNA samples from 100 patients and performed 50bp paired-end sequencing using the Illumina HiSeq platform. RNA sequencing libraries were prepared using the TruSeq kit (Illumina, Inc) with the following modification. Instead of purifying poly-A RNA using poly-dT primer beads, ribosomals removed RNA using the Ribominus kit (Ambion). All other steps were performed according to the manufacturer's protocols. RNAseq libraries were analyzed for QC and average size of inserts were approximately 200-300 bp. Samples were multiplexed into three samples per lane on the Illumina Version 3 Flowcells on the HiSeq2000 platform. Samples were sequenced at the Emory GRA Genomics Core Facility, Hudson Alpha Institute, and at the Southern California Genotyping Consortium (SCGC).

In total, approximately 490 billion base pairs (Gbp) of sequence were generated, of which 294 Gbp mapped uniquely to the human genome (build 19, hg19). In total, 5.874 billion mapped reads were obtained. The average number of mapped reads were 55.4M reads/sample, providing an average coverage of 34.2x for the human transcriptome.

FASTQ files generated from the Illumina HiSeq2000 were mapped to the human genome using TopHat software (version 2.0.8) and Bowtie (version 2.1.0), and Cufflinks software was used to generate fragment per kilobase per million reads (FPKM) values. Genes were filtered to determine if they were detected (defined as FPKM > 1) in each sample. Genes that were detected in 80% of BCR samples or 80% of non-BCR samples were retained, leaving a set of 5217 genes for further analysis. Duplicates from six patient samples were removed, and three patients had incomplete clinical data on PSA and stage, leaving 97 samples for biomarker analysis.

In order to construct a prediction model using the set of 5217 genes using 97 samples, the following analysis was performed: 1) standardized FPKM values were calculated for each gene (with mean 0 and sd 1); 2) apply the stability selection approach in combination with randomized lasso PH models for time to BCR to the set of 5217 genes to generate 1000 models on 1000 random sets of samples selected with replacement from the 97 available samples, and obtain a set of selected genes and their estimated coefficients (Table 4); 3) calculate predictive scores using the coefficients in Table 4 and standardized FPKM values; 4) calculate predictive scores with clinical variables by fitting a Cox PH model using the gene predictive scores and the set of clinical variables; and 5) subjects were divided into high and low risk groups based on the median predictive score and perform log rank test to compare BCR between the two risk groups with or without use of clinical variables (Figure 8). Survival prediction was greatly improved using our biomarkers in combination with clinical parameters relative to using clinical parameters alone (AUC increased from 0.74 to 0.97).

**Table 4: RNA Biomarkers of biochemical recurrence following prostatectomy identified by RNAseq analysis.**

| Symbol | Coefficient | % Models | Gene Name |
|---|---|---|---|
| SNORA20 | -0.507 | 40.1% | Small nucleolar RNA, H/ACA box 20 |
| HIST1H1C | 0.596 | 52.0% | Histone cluster 1, H1c |
| IFT57 | 0.34 | 71.8% | Intraflagellar transport 57 homolog |
| MIR663B | -0.941 | 92.8% | MicroRNA 663b |
| IGFBP3 | 0.489 | 44.7% | Insulin-like growth factor binding protein 3 |
| ITPR1 | -0.72 | 54.1% | Inositol 1,4,5-trisphosphate receptor, type 1 |
| PTN | -0.844 | 48.7% | Pleiotrophin |
| C15orf38 | 0.698 | 45.5% | Chromosome 15 open reading frame 38 |
| MARCH5 | -0.832 | 41.1% | Membrane-associated ring finger (C3HC4) 5 |
| EIF2D | 0.644 | 41.9% | Eukaryotic translation initiation factor 2D |
| RPL23AP53 | -0.334 | 46.9% | Ribosomal protein L23a pseudogene 53 |

To validate RNAseq data, TaqMan analysis was performed on several genes and correlation between TaqMan Fold change and RNAseq FPKM values were found. In addition, eleven biomarker genes were analyzed on the same independent data set from MSKCC from Taylor et al. The eleven biomarkers were significant at predicting BCR (p = 0.0006, AUC = 0.69). However, this analysis was limited by the fact that the dataset in Taylor et al. did not include expression for one of our biomarker genes, miR-663b, and thus this represents the lower bound of the ability of these biomarkers to predict outcome.

### Example 5

An alternative library preparation method was tested. A protocol was developed for robust RNAseq analysis of FFPE samples using the Ribominus kit (Ambion) to remove ribosomal RNA, followed by library preparation using the Illumina TruSeq kit. Multiplexing three samples per lane gave us adequate coverage for RNAseq analysis. Possibly due to the fact that we did not perform a poly-A selection step, a significant number of reads that mapped to gene introns, likely from partially processed mRNAs were observed. Moreover, the level of intronic reads was similar to that observed in RNAseq data derived from fresh frozen samples analyzed by The Cancer Genome Atlas project (TCGA). To validate and verify the accuracy of our RNAseq data, TaqMan analyses was performed on a few select genes and observed high correlation of TaqMan and RNAseq data for these genes (r2 = 0.80-0.97). In addition, expression of genes were analyzed that are typically involved in chromosomal translocations such as ERG, ETV1, ETV4, ETV5, and SPINK1. Mutually exclusive high levels of expression of ERG (45% of samples), ETV1 (6%), ETV4 (4%), and SPINK1 (10%) were observed. RNA from separate cores were prepared from the same six patients, and separate sequencing libraries were prepared on different days. Analysis of the fragment per kilobase of transcript per million mapped reads (FPKM) values from these replicate sequence analyses indicated very strong correlation (r2 = 0.70-0.96) for the 5,265 genes that were robustly detected in at least 80% of samples and used in our biomarker analyses. The pair of samples with the lowest correlation (UTPC034) had the greatest difference in number of mapped reads (18M vs. 112M), while the paired samples with the highest correlation (UTPC004) both had very deep coverage (94M and 101M mapped reads each). Differential gene expression analysis using DESeq indicated very few differentially expressed genes between replicate sequencing libraries (14 genes on average). For our biomarker analysis, the library with the higher number of mapped reads was used.

FASTQ files generated from the Illumina HiSeq2000 were mapped to the human genome using TopHat software (version 2.0.8) and Bowtie (version 2.1.0), and Cufflinks software was used to generate FPKM values. Genes were filtered to determine if they were detected (defined as FPKM > 1) in each sample. The distribution of detected genes were analyzed, and a peak of genes that were detected in 80% or more of the samples were observed. To avoid excluding those genes that were not expressed in one of the two groups, genes detected in either 80% of BCR or 80% of non-BCR samples were retained. Genes that were detected in 80% of BCR samples or 80% of non-BCR samples included a set of 5265 protein-coding or non-coding genes for further analysis. Duplicates from six patient samples were removed, and three patients had incomplete clinical data on PSA and pathologic stage, leaving 97 samples for biomarker analysis.

**Table 5: Twenty Four Biomarkers of biochemical recurrence following prostatectomy identified by RNAseq analysis**

| Selected Genes Coefficient | | |
|---|---|---|
| BTG2 | -0.08831 | B-cell Translocation Gene 2 |
| CDC37L1 | -0.13272 | Cell Division Cycle 37-Like 1 |
| COL15A1 | 0.007957 | Collagen, Type XV, Alpha 1 |
| COL3A1 | 0.27692 | Collagen, Type III, Alpha 1 |
| EIF2D | 0.403303 | Eukaryotic translation initiation factor 2D |
| FDPS | 0.019765 | Farnesyl diphosphate synthase |
| HIST1H1C | 0.088821 | Histone cluster 1, H1c |
| HIST1H2BG | 0.067189 | Histone cluster 1, H2bg |
| IFT57 | 0.274924 | Intraflagellar transport 57 homolog |
| IGFBP3 | 0.076922 | Insulin-like growth factor binding protein 3 |
| ITPR1 | -0.27849 | Inositol 1,4,5-trisphosphate receptor, type 1 |
| LBH | 0.098016 | Limb Bud and Heart Development |
| LOC284801 | 0.022612 | |
| MARCH5 | -0.25456 | Membrane-associated ring finger (C3HC4) 5 |
| MED4 | -0.14 | Mediator Complex Subunit 4 |
| MEMO1 | 0.116525 | Mediator of Cell Motility 1 |
| MXI1 | -0.03462 | MAX interactor 1, dimerization protein |
| PTN | -0.16271 | Pleiotrophin |
| RPL23AP53 | -0.12988 | Ribosomal protein L23a pseudogene 53 |
| SACM1L | -0.02007 | SAC1 suppressor of actin mutations 1-like |
| SIRT1 | -0.08227 | Sirtuin 1 |
| SNORA20 | -0.10131 | Small nucleolar RNA, H/ACA box 20 |
| SRSF3 | -0.02622 | Serine/Arginine-rich Splicing Factor 3 |
| SYNM | -0.0574 | Synemin, Intermediate Filament Protein |

Using the set of 5,265 genes from 97 samples, a 24-gene prediction model was built using a pre-selection step and a lasso Cox PH model and the final prediction model was built to include the predictive score based on this panel of 24 markers as well as the relevant clinical biomarkers including pathologic stage, PSA, Gleason score, surgical margin status, and age. For comparison, a prediction model was built using only clinical information, namely, pathologic stage, PSA, Gleason score, surgical margin status, and age, through fitting a Cox PH model. Log-rank tests were performed to compare BCR between the low risk (good score) and high risk (poor score) groups with or without use of clinical variables (Figure 10). Kaplan-Meier analysis (Figure 10A and 10C) demonstrated that these markers (Table 5) could significantly discriminate patients at higher and lower risk of recurrence by the log-rank test (p = 1.45e-21) in the training data, more significant than using clinical variables alone (p = 5.39e-8). The improvements of the full model over the model using only clinical parameters in prediction as measured by IDI, NRI, and median improvement in risk score for censored survival outcomes were all statistically significant.

To validate this panel of biomarkers, an independent gene expression microarray study with data from 140 prostate cancer patients was identified. Using the data from Taylor et al., the final prediction models obtained from the training phase were evaluated. Each prediction model from the training phase was used to generate a predictive score for each subject in the testing data set, and subjects were subsequently divided into the high and low risk groups using the median predictive score. Log-rank tests were performed to compare time to BCR between the high (poor score) and low (good score) risk groups. The prediction model based on clinical variables alone was tested (Figure 10B), showing significant discriminative performance in the validation data as well (p = 2.85e-3). In addition, it was observed that the full panel including RNA biomarkers and clinical variables was significantly prognostic in this independent validation set (p = 7.87e-5, Figure 10D).

A direct comparison of the 24 biomarker genes in Table 5 was performed with a set of 31 cell cycle progression genes. See Cuzick et al. Prognostic value of an RNA expression signature derived from cell cycle proliferation genes in patients with prostate cancer: a retrospective study. Lancet Oncol. 2011;12:245-55. The panel in Cuzick (Myriad panel) performed less well in our the dataset (p = 4.94e-8) than the biomarker panel in Table 5 at a level similar to clinical parameters alone. In the validation set from Taylor et al., the Table 5 panel of biomarker genes outperformed the 31 cell cycle progression genes which had less significant p-value for the log-rank test (p = 1.4e-4, Figure 10F). Furthermore, analysis for IDI, NRI and median improvement in risk score demonstrated that the Table 5 panel was statistically significantly better in prediction of recurrence than the Myriad panel.

Table 6 shows a comparison of prediction performance of the full model including the RNA biomarkers and clinical parameters with other models that include clinical variables alone, RNA biomarkers alone, or the MYRIAD model in terms of integrated discrimination improvement (IDI), net reclassification improvement (NRI) and median improvement in risk score for censored survival outcome. A positive value in IDI or NRI indicates an improvement over the second model. Significant p-values (bold font) indicate a significant improvement of the full model over other models in prediction of BCR.

**Table 6 Comparison of Prediction Performance of Biomarker Panel in Table 5**

| | | **IDI** | **p value** | **NRI** | **p value** | **median improvement in risk score** | **p value** |
|---|---|---|---|---|---|---|---|
| **Training Set** | Full Model vs Clinical Variables only | 0.469 | **0.024** | 0.875 | **0.022** | 0.394 | **0.004** |
| **Validation Set** | Full Model vs RNA Biomarkers only | 0.218 | **0.036** | 0.718 | **0.043** | 0.2 | **0.005** |
| | Model vs MYRIAD Model | 0.676 | **0.03** | 0.875 | **0.045** | 0.634 | **<0.001** |
| | Full Model vs Clinical Variables only | 0.699 | **0.019** | 0.669 | **0.042** | 0.678 | **<0.001** |
| | Full Model vs RNA Biomarkers only | 0.005 | 0.643 | -0.439 | 0.246 | -0.002 | 0.986 |
| | Full Model vs MYRIAD Model | 0.601 | **0.027** | 0.625 | **0.051** | 0.652 | **<0.001** |

## Claims

1. A method of determining the risk of recurrence of prostate cancer in a subject, comprising
analyzing a prostatectomy sample from a subject to determine RNA levels of biomarkers BTG2, CDC37L1, COL15A1, COL3A1, EIF2D, FDPS, HIST1H1C, HIST1H2BG, IFT57, IGFBP3, ITPR1, LBH, LOC284801, MARCH5, MED4, MEMO1, MXI1, PTN, RPL23AP53, SACM1L, SIRT1, SNORA20, SRSF3, and SYNM in the sample, and
comparing the levels of all of the biomarkers in the sample to prostate cancer-positive and/or prostate cancer-negative reference levels of the biomarkers, wherein
levels of the biomarkers in the sample matching the prostate cancer-positive reference levels, or being differentially present compared to the prostate cancer-negative reference levels, are indicative of the subject being predisposed to recurrence of prostate cancer, and wherein levels of the biomarkers in the sample matching the prostate cancer-negative reference levels, or being differentially present compared to the cancer-positive reference levels, are indicative of the subject not being predisposed to recurrence of prostate cancer.

2. The method of Claim 1, further comprising recording the measurements or comparisons of the biomarkers on a computer readable medium.

3. A kit comprising nucleic acids configured to bind 24 RNA biomarkers for cancer, wherein the RNA biomarkers are BTG2, CDC37L1, COL15A1, COL3A1, EIF2D, FDPS, HIST1H1C, HIST1H2BG, IFT57, IGFBP3, ITPR1, LBH, LOC284801, MARCH5, MED4, MEMO1, MXI1, PTN, RPL23AP53, SACM1L, SIRT1, SNORA20, SRSF3, and SYNM, and wherein the nucleic acids configured to bind the RNA biomarkers are covalently attached to an array.

4. The kit of Claim 3, further comprising one or more or all of the components selected from oligonucleotides or pairs configured to bind for copying predetermined nucleic acid sequences associated with the biomarkers, adaptor oligonucleotides configured to bind to oligonucleotide pairs, adaptor oligonucleotides configured to bind or hybridize to nucleic acids attached to an array, ligase, circularization ligase, polymerase, a mix of deoxynucleotides, a biotinylated nucleotide, and a streptavidin bead.

## Patentansprüche

1. Verfahren zur Bestimmung des Risikos eines erneuten Auftretens von Prostatakrebs bei einem Patienten, umfassend
Analyse einer Prostatektomieprobe von einem Subjekt zur Bestimmung der RNA-Spiegel der Biomarker BTG2, CDC37L1, COL15A1, COL3A1, EIF2D, FDPS, HIST1H1C, HIST1H2BG, IFT57, IGFBP3, ITPR1, LBH, LOC284801, MARCH5, MED4, MEMO1, MXI1, PTN, RPL23AP53, SACM1L, SIRT1, SNORA20, SRSF3, und SYNM in der Probe und
Vergleichen der Konzentrationen aller Biomarker in der Probe mit Prostatakrebs-positiven und/oder Prostatakrebs-negativen Referenzkonzentrationen der Biomarker, wobei
die Konzentrationen der Biomarker in der Probe, die mit den Prostatakrebs-positiven Referenzniveaus übereinstimmen oder im Vergleich zu den Prostatakrebs-negativen Referenzniveaus unterschiedliche Werte aufweisen, indikativ dafür sind, dass das Subjekt für ein Wiederauftreten von Prostatakrebs prädisponiert ist, und wobei die Konzentrationen der Biomarker in der Probe, die mit den Prostatakrebs-negativen Referenzniveaus übereinstimmen, oder die im Vergleich zu den Krebs-positiven Referenzniveaus unterschiedliche Werte aufweisen, indikativ dafür sind, dass das Subjekt nicht für ein Wiederauftreten von Prostatakrebs prädisponiert ist.

2. Verfahren nach Anspruch 1, ferner umfassend das Aufzeichnen der Messungen oder Vergleiche der Biomarker auf einem computerlesbaren Medium.

3. Kit, umfassend Nukleinsäuren, die konfiguriert sind, um 24 RNA-Biomarker für Krebs zu binden, wobei die RNA-Biomarker BTG2, CDC37L1, COL15A1, COL3A1, EIF2D, FDPS, HIST1H1C, HIST1H2BG, IFT57, IGFBP3, ITPR1, LBH, LOC284801, MARCH5, MED4, MEMO1, MXI1, PTN, RPL23AP53, SACM1L, SIRT1, SNORA20, SRSF3 und SYNM sind, und wobei die zur Bindung der RNA-Biomarker konfigurierten Nukleinsäuren kovalent an ein Array gebunden sind.

4. Kit nach Anspruch 3, ferner umfassend eine oder mehrere oder alle Komponenten, ausgewählt aus Oligonukleotiden oder Paaren, die konfiguriert sind, um zum Nachbilden vorgegebene Nukleinsäuresequenzen, die den Biomarkern zugeordnet sind, zu binden, Adapter-Oligonukleotide, die konfiguriert sind, um sich an Oligonukleotidpaare zu binden, Adapter-Oligonukleotide, die konfiguriert sind, um an Nukleinsäuren zu binden oder zu hybridisieren, die an ein Array, eine Ligase, eine Zirkulationsligase, eine Polymerase, eine Mischung aus Desoxynukleotiden, ein biotinyliertes Nukleotid und eine Streptavidinperle gebunden sind.

## Revendications

1. Méthode de détermination du risque de récurrence d'un cancer de la prostate chez un sujet, comprenant l'analyse d'un échantillon de prostatectomie prélevé chez un sujet afin de déterminer les taux d'ARN des biomarqueurs de BTG2, CDC37L1, COL15A1, COL3A1, EIF2D, FDPS, HIST1H1C, HIST1H2BG, IFT57, IGFBP3, ITPR1, LBH, LOC284801, MARCH5, MED4, MEMO1, MXI1, PTN, RPL23AP53, SACM1L, SIRT1, SNORA20, SRSF3, et SYNM dans l'échantillon, et la comparaison des taux de tous les biomarqueurs dans l'échantillon avec des taux de référence, positifs pour le cancer de la prostate et/ou négatifs pour le cancer de la prostate, des biomarqueurs, où les taux des biomarqueurs dans l'échantillon correspondant aux taux de référence positifs pour le cancer de la prostate, ou étant différentiellement présents par rapport aux taux de référence négatifs pour le cancer de la prostate, constituent une indication du fait que le sujet est prédisposé à une récurrence du cancer de la prostate, et où les taux des biomarqueurs dans l'échantillon correspondant aux taux de référence négatifs pour le cancer de la prostate, ou étant différentiellement présents par rapport aux taux de référence positifs pour le cancer, constituent une indication du fait que le sujet n'est pas prédisposé à une récurrence du cancer de la prostate.

2. Méthode selon la revendication 1, comprenant en outre l'enregistrement des mesures ou des comparaisons des biomarqueurs sur un moyen lisible par un ordinateur.

3. Kit comprenant des acides nucléiques configurés pour fixer 24 biomarqueurs d'ARN pour le cancer, où les biomarqueurs d'ARN sont BTG2, CDC37L1, COL15A1, COL3A1, EIF2D, FDPS, HIST1H1C, HIST1H2BG, IFT57, IGFBP3, ITPR1, LBH, LOC284801, MARCH5, MED4, MEMO1, MXI1, PTN, RPL23AP53, SACM1L, SIRT1, SNORA20, SRSF3, et SYNM, et où les acides nucléiques configurés pour fixer les biomarqueurs d'ARN sont fixés de manière covalente à un réseau.

4. Kit selon la revendication 3, comprenant en outre un ou plusieurs, ou l'ensemble, des composants choisis parmi des oligonucléotides ou des paires configuré(e)s pour se fixer afin de copier des séquences d'acide nucléique prédéterminées associées aux biomarqueurs, des oligonucléotides adaptateurs configurés pour se fixer à des paires oligonucléotidiques, des oligonucléotides adaptateurs configurés pour se fixer ou s'hybrider avec des acides nucléiques fixés à un réseau, une ligase, une ligase de circularisation, une polymérase, un mélange de désoxynucléotides, un nucléotide biotinylé, et des billes de streptavidine.
